# EUROPÄISCHE PATENTSCHRIFT

(11) **EP 3 875 045 B1**
(45) Veröffentlichungstag und Bekanntmachung des Hinweises auf die Patenterteilung: **27.09.2023**
(21) Anmeldenummer: 21159194.6
(22) Anmeldetag: 25.02.2021
(51) Int. Cl.: A61B 17/22, B06B 3/00

(54) **LITHOTRIPSIEVORRICHTUNG**
LITHOTRIPSY DEVICE
DISPOSITIF DE LITHOTRIPSIE

(30) Priorität: 02.03.2020 DE 102020105457
(43) Veröffentlichungstag der Anmeldung: 08.09.2021
(73) Patentinhaber: Karl Storz SE & Co. KG, 78532 Tuttlingen (DE)
(72) Erfinder: Glöggler, Bernhard, 78532 Tuttlingen (DE); Hinding, Thomas, 78532 Tuttlingen (DE); Huber, Florian, 78532 Tuttlingen (DE)

(56) Entgegenhaltungen:
- US-A- 4 516 398
- US-A- 5 123 903
- US-A1- 2010 010 395
- US-A1- 2010 056 986

## Beschreibung

### Stand der Technik

Die Erfindung betrifft eine Lithotripsievorrichtung gemäß dem Oberbegriff des Anspruchs 1. Ferner kann die Erfindung einen Lithotripter mit wenigstens einer solchen Lithotripsievorrichtung, einen Bausatz für wenigstens einen solchen Lithotripter, ein Lithotripsiesystem mit wenigstens eines solchen Lithotripters, sowie ein Verfahren zur Herstellung und/oder Betrieb wenigstens einer solchen Lithotripsievorrichtung betreffen.

Die US 2010/056986 A1 offenbart eine Vorrichtung für die Phakoemulsifikation, also das Zerkleinern und Absaugen des Linsenkerns des Auges mittels einer mit Ultraschall angeregten Kanüle und das anschließende Absaugen der Trümmer mittels einer Saugspülvorrichtung, wobei der Absaugungskanal zentral liegt und der Irrigationskanal, über welchen die Irrigationsflüssigkeit an die Außenseite der Kanüle abgegeben wird und an dieser bis zum Auge herunter rinnt, zwischen Horn und Kappe verläuft und zur Kühlung dient. Es ist physikalisch nicht möglich diese Vorrichtung invertiert zu betreiben, also den Irrigationskanal zur Abführung des Irrigats zu verwenden, da dafür das Irrigat an der Außenseite der Kanüle hochkriechen müsste. Demzufolge ist die Fließrichtung für die technische Wirkung entscheidend.

Aus der EP 1 163 883 B2 ist bereits ein Lithotripter bekannt, welcher zur Beaufschlagung vor Konkrementen mit Ultraschallwellen eingerichtet ist, und ein Ultraschallhorn, welches zumindest zur Fokussierung und/oder Übertragung der Ultraschallwellen auf die Sonotrode eingerichtet ist, umfasst. Ferner weist die Lithotripsievorrichtung ein sich durch die gesamte Sonotrode und Ultraschallhorn hindurch erstreckenden Irrigationskanal auf, welcher zur Leitung eines Irrigats eingerichtet ist. Der Irrigationskanal ist dabei zu einem Abführen des Irrigats innerhalb des Ultraschallhorns mit wenigstens einem Abführungskanal fluidtechnisch verbunden. Der Lithotripter weist zudem eine Kappe auf, welche die Sonotrode umgibt.

Die Aufgabe der Erfindung besteht insbesondere darin, eine gattungsgemäße Vorrichtung mit verbesserten Eigenschaften hinsichtlich einer Kühlung, insbesondere in Kombination mit einer effizienten, kompakten und/oder sicheren Bauweise bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Lithotripsievorrichtung, insbesondere einer intrakorporalen Lithotripsievorrichtung, mit wenigstens einer Sonotrode, welche zur Beaufschlagung von Konkrementen zumindest mit Ultraschallwellen eingerichtet ist, mit wenigstens einem Ultraschallhorn, welches zumindest zur Fokussierung und/oder Übertragung von Ultraschallwellen auf die Sonotrode eingerichtet ist, mit wenigstens einem Irrigationskanal, welcher zur Leitung eines Irrigats eingerichtet ist und welcher sich zumindest teilweise durch die Sonotrode und/oder das Ultraschallhorn erstreckt, mit wenigstens einer Kappe, welche die Sonotrode und/oder das Ultraschallhorn zumindest teilweise umgebend angeordnet ist, sowie mit wenigstens einem Abführungskanal, welcher fluidtechnisch zu einem Abführen des Irrigats mit dem Irrigationskanal verbunden ist.

Es wird vorgeschlagen, dass der Abführungskanal wenigstens teilweise von einem zwischen der Kappe und der Sonotrode und/oder dem Ultraschallhorn eingeschlossenen Spalt ausgebildet ist und zu einer Kühlung der Sonotrode und/oder des Ultraschallhorns mittels des abgeführten Irrigats eingerichtet ist.

Hierdurch kann vorteilhaft eine Kühlung verbessert werden. Durch sich entlang der Sonotrode und dem Ultraschallhorn ausbreitende Ultraschallwellen kann es in einem Betriebszustand der Lithotripsievorrichtung zu einer Erhitzung gegenüber einem Ruhezustand der Lithotripsievorrichtung kommen, welche durch die vorliegende Ausgestaltung verringert werden kann, da Sonotrode und/oder das Ultraschallhorn durch das Irrigat gekühlt werden kann. Weiter vorteilhaft kann eine Bauweise der Lithotripsievorrichtung vereinfacht werden, da beispielsweise eine Bauweise und/oder Anordnung des Ultraschallgenerator zur Bereitstellung der Ultraschallwellen nicht durch das Vorhandensein eines Abführungskanals eingeschränkt ist. Weiter vorteilhaft kann eine Sicherheit der Lithotripsievorrichtung erhöht werden, da insbesondere das Irrigat von elektrischen Bauteilen, wie beispielsweise dem Ultraschallgenerator baulich getrennt werden kann. Somit kann vorteilhaft vermieden werden, dass in etwas in Fall eines Kurzschlusses zwischen solchen Bauteilen und dem Irrigat Strom auf einen Patienten übertragen werden kann.

Unter einer "Lithotripsievorrichtung" soll insbesondere ein, vorzugsweise funktionsfähiger Bestandteil, insbesondere eine Unterbaugruppe und/oder eine Konstruktions- und/oder eine Funktionskomponente eines Lithotripters verstanden werden. Vorzugsweise kann die Lithotripsievorrichtung den Lithotripter zumindest teilweise, vorzugsweise zumindest zu einem Großteil und besonders bevorzugt vollständig ausbilden. Die Lithotripsievorrichtung ist zu einer Zertrümmerung von Konkrement eingerichtet. Unter "Konkrementen" sollen insbesondere sich im Körper durch Abscheidung entstehende feste Gebilde verstanden werden, welche zumindest teilweise, vorzugsweise zumindest zu einem Großteil und besonders bevorzugt vollständig aus Salzen bestehen, wie beispielsweise Nierensteine, Gallensteine, Harnsteine, Speichelsteine oder dergleichen. Unter "eingerichtet" soll insbesondere speziell programmiert, vorgesehen, ausgelegt und/oder ausgestattet verstanden werden. Darunter, dass ein Objekt zu einer bestimmten Funktion eingerichtet ist, soll insbesondere verstanden werden, dass das Objekt diese bestimmte Funktion in zumindest einem Anwendungs- und/oder Betriebszustand erfüllt und/oder ausführt. Die Lithotripsievorrichtung ist vorzugsweise als eine intrakorporale Lithotripsievorrichtung ausgebildet, welche dazu ausgebildet ist, zumindest teilweise und vorzugsweise zumindest zu einem Großteil in eine insbesondere künstliche und/oder natürliche Öffnung, insbesondere Körperöffnung, eingeführt zu werden, um sich dort angesammeltes Konkrement zu zertrümmern und/oder zu entfernen. Unter dem Ausdruck "zumindest zu einem Großteil" soll dabei insbesondere zumindest zu 55 %, vorzugsweise zumindest zu 65 %, bevorzugt zumindest zu 75 %, besonders bevorzugt zumindest zu 85 % und ganz besonders bevorzugt zumindest zu 95 %, sowie vorteilhaft vollständig verstanden werden und zwar insbesondere mit Bezug auf ein Volumen und/oder eine Masse eines Objekts. Zu einer Zertrümmerung von Konkrementen nutzt die Lithotripsievorrichtung wenigstens Ultraschallwellen. Unter "Ultraschallwellen" sollen insbesondere Schallwellen oberhalb eines Hörfrequenzbereichs des Menschen verstanden werden. Im vorliegenden Fall weisen die Ultraschallwellen insbesondere eine Ultraschallfrequenz von zumindest 10 kHz, vorzugsweise zumindest 15 kHz und besonders bevorzugt zumindest 20 kHz und/oder von höchstens 100 kHz, vorzugsweise von höchstens 80 kHz und besonders bevorzugt von höchstens 56 kHz auf. Ganz besonders bevorzugt beträgt die Ultraschallfrequenz zwischen 22 kHz und 55 kHz. Zur Bereitstellung von Ultraschallwellen weist die Lithotripsievorrichtung wenigstens einen Ultraschallgenerator auf. Der Ultraschallgenerator ist insbesondere dabei dazu eingerichtet, Ultraschallwellen zu erzeugen und vorzugsweise weitere Komponenten bereitzustellen. Der Ultraschallgenerator weist insbesondere zumindest einen, vorzugsweise zumindest zwei, besonders bevorzugt zumindest drei und ganz besonders bevorzugt eine Vielzahl von Ultraschallaktoren auf, welcher/welche insbesondere als ein Piezoaktor/Piezoaktoren ausgebildet ist/sind. Insbesondere für den Fall, dass der Ultraschallgenerator zumindest zwei Ultraschallaktoren umfasst liegen diese zumindest mittelbar aneinander an, um vorteilhaft eine Intensität des erzeugten Ultraschalls zu erhöhen. Der Ultraschallgenerator ist insbesondere mit dem Ultraschallhorn verbunden. Das Ultraschallhorn weist insbesondere eine sich vorzugsweise konkav verjüngende Zylinderform auf. Das Ultraschallhorn kann beispielsweise zumindest teilweise, vorzugsweise zumindest zu einem Großteil und besonders bevorzugt vollständig aus Metall, wie beispielsweise Eisen, Titan, Stahl oder einer Metalllegierung ausgebildet sein.

Die Sonotrode ist dazu ausgebildet bei einem Lithotripsieverfahren das Konkrement zumindest mittelbar oder unmittelbar zu kontaktieren. Ferner ist die Sonotrode dazu ausgebildet, in eine Körperöffnung eingeführt zu werden. Unter einer "Sonotrode" soll insbesondere ein längliches Bauteil der Lithotripsievorrichtung verstanden werden. Unter einem "länglichen Bauteil" soll insbesondere ein Bauteil verstanden werden, dessen Haupterstreckung zumindest um einen Faktor fünf, vorzugsweise zumindest um einen Faktor zehn und besonders bevorzugt zumindest um einen Faktor zwanzig größer ist als eine Erstreckung des Bauteils senkrecht zur Haupterstreckungsrichtung, also insbesondere einem Durchmesser des Bauteils. Die Sonotrode weist insbesondere einen Durchmesser von zumindest 1 mm, vorzugsweise zumindest 2 mm und besonders bevorzugt zumindest 4 mm auf. Ferner weist der Schaft einen Durchmesser von höchstens 20 mm, vorzugsweise höchstens 18 mm und besonders bevorzugt von höchstens 16 mm auf. Unter "zumindest im Wesentlichen" soll insbesondere eine maximale Abweichung eines Wertes von höchstens 10 %, vorzugsweise höchstens 5% und besonders bevorzugt höchstens 2 % verstanden werden. Die Sonotrode definiert insbesondere einen distalen Abschnitt der Lithotripsievorrichtung. Unter "distal" soll insbesondere bei einer Bedienung einem Patienten zugewandt und/oder einem Bediener abgewandt verstanden werden. Ferner kann unter distal der Gegensatz zu proximal verstanden werden. Unter "proximal" soll insbesondere bei einer Bedienung einem Patienten abgewandt und/oder einem Bediener zugewandt verstanden werden.

Die Sonotrode ist insbesondere mit dem Ultraschallhorn wirkverbunden. Beispielsweise sind das Ultraschallhorn und die Sonotrode kraft- und/oder formschlüssig miteinander verbunden. Unter einer "kraft- und/oder formschlüssigen Verbindung" ist insbesondere eine Verbindung zu verstehen, welche vorzugsweise werkzeuglos und/oder zerstörungsfrei lösbar ist, wobei eine Haltekraft zwischen zwei Objekten über eine solche Verbindung verbundener Objekte beispielsweise über einen geometrischen Eingriff ineinander und/oder über eine Reibungskraft, die vorzugsweise zwischen den Objekten wirkt, zustande kommt. Vorzugsweise handelt es sich bei der Verbindung, um eine Flanschverbindung. Ganz besonders bevorzugt handelt es sich bei der Verbindung um eine Schraubverbindung mittels eines Gewindes. Ferner könnte die Verbindung zwischen Sonotrode und Ultraschallhorn stoffschlüssig sein. Unter einer "stoffschlüssigen Verbindung" soll insbesondere verstanden werden, dass die Objekte durch atomare oder molekulare Kräfte zusammengehalten werden, wie beispielsweise beim Löten, Schweißen, Kleben und/oder Vulkanisieren. Ferner ist denkbar, dass das Ultraschallhorn und die Sonotrode einstückig miteinander ausgebildet/verbunden sind. Ferner könnte die Verbindung zwischen Sonotrode und das Ultraschallhorn zumindest teilweise einstückig ausgebildet/verbunden sein. Darunter, dass "ein Objekt und ein weiteres Objekt zumindest teilweise einstückig ausgebildet/verbunden sind", soll insbesondere verstanden werden, dass zumindest ein Element und/oder Teil des Objekts und zumindest ein Element und/oder Teil des weiteren Objekts einteilig ausgebildet/verbunden sind. Unter "einstückig" soll insbesondere zumindest stoffschlüssig verbunden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling. Alternativ oder zusätzlich könnten die Sonotrode und/oder das Ultraschallhorn durch ein generatives Verfahren hergestellt sein, wie beispielsweise 3D-Druck, insbesondere durch Laserschweißen und/oder Lasersintern. Die Sonotrode ist beispielsweise rohrförmig ausgebildet. Die Sonotrode ist insbesondere zumindest teilweise, vorzugsweise zumindest zu einem Großteil und besonders bevorzugt vollständig von Metall, wie beispielsweise Eisen, Titan, Stahl oder einer Metalllegierung ausgebildet.

Der Irrigationskanal ist insbesondere als ein röhrenförmiger Kanal ausgebildet. Unter einem "Kanal", wie beispielsweise der Irrigationskanal oder der Abführungskanal, soll insbesondere ein Objekt verstanden werden, das zumindest teilweise zu einer Führung eines Fluidstroms vorgesehen ist, und die insbesondere den Fluidstrom, insbesondere zumindest im Wesentlichen senkrecht zu einer Hauptfluidstromrichtung begrenzt und/oder unmittelbar zumindest teilweise, vorzugsweise auf wenigstens drei Seiten, besonders bevorzugt vollständig, vorteilhaft von genau vier Seiten und besonders bevorzugt zumindest entlang einer Umfangsrichtung, vollständig umschließt. Unter einer "Hauptfluidstromrichtung" soll insbesondere eine effektive Strömungsrichtung eines Fluids, vorzugsweise eine über einen Bereich gemittelte Fließrichtung eines Fluids, verstanden werden. Unter "zumindest im Wesentlichen senkrecht" soll hier insbesondere eine Ausrichtung einer Richtung relativ zu einer Bezugsrichtung, insbesondere in einer Ebene, verstanden werden, wobei die Richtung und die Bezugsrichtung einen Winkel von 90°, insbesondere unter Berücksichtigung einer maximalen Abweichung von kleiner als 8°, vorteilhaft von kleiner als 5° und besonders vorteilhaft von kleiner als 2° einschließt. Der Irrigationskanal erstreckt sich insbesondere zumindest zu einem Großteil durch die Sonotrode und/oder das Ultraschallhorn. Vorzugsweise erstreckt sich der Irrigationskanal nicht vollständig durch die gesamte Sonotrode und/oder das Ultraschallhorn. Eine Haupterstreckungsrichtung des Irrigationskanal ist insbesondere zumindest im Wesentlichen parallel zu einer Haupterstreckungsrichtung der Sonotrode und/oder des Ultraschallhorns. Unter "zumindest im Wesentlichen parallel" soll hier insbesondere eine Ausrichtung einer Richtung relativ zu einer Bezugsrichtung, insbesondere in einer Ebene, verstanden werden, wobei die Richtung und die Bezugsrichtung einen Winkel von 0° insbesondere unter Berücksichtigung einer maximalen Abweichung von kleiner als 8°, vorteilhaft von kleiner als 5° und besonders vorteilhaft von kleiner als 2° einschließt. Unter einer "Haupterstreckungsrichtung" eines Objekts soll dabei insbesondere eine Richtung verstanden werden, welche parallel zu einer längsten Kante eines kleinsten gedachten Quaders verläuft, welcher das Objekt gerade noch vollständig umschließt. Der Irrigationskanal verläuft zumindest teilweise und vorzugsweise zumindest zu einem Großteil koaxial in der Sonotrode und/oder dem Ultraschallhorn. Um Irrigat mit dem Irrigationskanal auszutauschen weist die Sonotrode insbesondere wenigstens eine distalseitige Öffnung auf, welche fluidtechnisch mit dem Irrigationskanal verbunden ist.

Ferner weist die Lithotripsievorrichtung insbesondere ein Gehäuse auf, welches vorzugsweise hohlzylinderförmig ausgebildet ist. Das Gehäuse definiert einen proximalen Abschnitt der Lithotripsievorrichtung. In dem Gehäuse können weitere Komponenten der Lithotripsievorrichtung angeordnet sein. Ferner ist insbesondere das Ultraschallhorn zumindest teilweise in dem Gehäuse angeordnet.

Es ist denkbar, dass die Kappe in/an dem Gehäuse zumindest teilweise angeordnet ist. Alternativ könnte die Kappe auch einstückig mit dem Gehäuse ausgebildet sein. Die Kappe umgibt insbesondere zumindest teilweise, vorzugsweise zumindest zu einem Großteil die Sonotrode und zwar vorzugsweise entlang eines Umfangs der Sonotrode. Die Kappe umgibt insbesondere zumindest teilweise, vorzugsweise zumindest zu einem Großteil das Ultraschallhorn zumindest teilweise und zwar vorzugsweise entlang eines Umfangs des Ultraschallhorns. Die Kappe umgibt insbesondere zumindest teilweise, vorzugsweise zumindest zu einem Großteil das Gehäuse zumindest teilweise und zwar vorzugsweise entlang eines Umfangs des Gehäuses. Die Kappe kann beispielsweise aus einem Kunststoff und/oder einem Metall ausgebildet sein. Bevorzugt ist die Kappe einstückig ausgebildet. Die Kappe ist insbesondere zumindest abschnittsweise rohrförmig, hohlkegelförmig und/oder trichterförmig ausgebildet. Die Kappe weist insbesondere einen distalen Abschnitt auf, welcher rohrförmig ist. Ferner weist die Kappe insbesondere einen proximalen Abschnitt auf, welcher rohrförmig ist. Zudem weist die Kappe insbesondere wenigstens einen mittleren Abschnitt auf, welcher trichterförmig ist. Der mittlere Abschnitt liegt insbesondere zwischen dem proximalen Abschnitt und dem distalen Abschnitt.

Der Abführungskanal ist eingerichtet, so dass ein durch diesen fließendes Irrigat die Sonotrode und/oder das Ultraschallhorn zumindest teilweise umspült und zwar insbesondere unmittelbar oder mittelbar, wobei das Irrigat vorzugsweise in Kontakt mit der Sonotrode und/oder dem Ultraschallhorn kommt. Der Abführungskanal bzw. das eingeschlossene Volumen des Abführungskanals ist wenigstens abschnittsweise rohrförmig und/oder trichterförmig ausgebildet. Der Abführungskanal ist insbesondere zumindest dazu eingerichtet das Irrigat des Irrigationskanals abzuführen. Der Abführungskanal umgibt insbesondere entlang eines Umfangs zumindest teilweise, vorzugsweise zumindest zu einem Großteil und besonders bevorzugt vollständig de Sonotrode und/oder das Ultraschallhorn. Die Kappe und die Sonotrode und/oder das Ultraschallhorn bilden vorzugsweise zumindest teilweise, vorzugsweise zumindest zu einem Großteil den Abführungskanal aus. Zur Förderung des Irrigats, insbesondere entlang des Irrigationskanals und/oder oder eines Abführungskanals kann eine Fördereinrichtung, wie beispielsweise eine Pumpe, vorgehsehen sein. Die Fördereinrichtung ist vorteilhaft separat von der Lithotripsievorrichtung ausgebildet. Beispielsweise kann die Fördereinrichtung Teil eines gemeinsamen Lithotripsiesystems mit einem die Lithotripsievorrichtung umfassenden Lithotripters sein. Auch könnte die Fördereinrichtung in ein Steuergerät des Lithotripsiesystems integriert sein.

Die Lithotripsievorrichtung kann ferner wenigstens ein Projektil aufweise, welches dazu eingerichtet ist, zusätzlich zu den Ultraschallwellen, das Ultraschallhorn und/oder die Sonotrode mit Stößen zu beaufschlagen. Insbesondere ist das Projektil dazu eingerichtet, bei einer Bewegung die Sonotrode mit einer Stoßfrequenz zu beaufschlagen, welche wesentlich kleiner ist als die Ultraschallfrequenz der Ultraschallwellen. Es soll unter "wesentlich kleiner" zumindest insbesondere um einen Faktor 10, vorzugsweise zumindest um einen Faktor 100 und besonders bevorzugt zumindest um einen Faktor 1000 geringer verstanden werden. Das Projektil weist insbesondere eine Form eines Rings, eines Zylinders, insbesondere eines Hohlzylinders auf. Vorzugsweise besteht das Projektil zumindest teilweise aus Metall, wie beispielsweise Stahl, insbesondere gehärteter Stahl oder Federstahl. Besonders bevorzugt weist das Projektil eine Beschichtung auf, wie insbesondere eine Härtungsbeschichtung, Gleitbeschichtung, eine Antihaftbeschichtung, eine Korrosionsbeschichtung oder dergleichen. Vorzugsweise ist die Antihaft-, Gleit, und/oder Korrosionsbeschichtung auf in einem Betriebszustand an weiteren Flächen reibenden Flächenabschnitten, wie beispielsweise einer Mantelfläche, des Projektils angeordnet. Bei der Antihaft-, Gleit, und/oder Korrosionsbeschichtung kann es sich beispielsweise um eine PTFE-, Keramik-, Kohlenstoff-Beschichtung oder dergleichen handeln. Vorzugsweise ist die Härtungsbeschichtung auf in einem Betriebszustand auf weitere Flächen stoßenden Flächenabschnitten, wie beispielsweise einer Deckfläche des Projektils angeordnet. Bei der Härtungsbeschichtung kann es sich beispielsweise um eine Metall-Legierung, insbesondere Titanlegierung handeln.

Das Projektil könnte entlang eines Führungskanal der Lithotripsievorrichtung beweglich gelagert sein. Der Führungskanal ist insbesondere wenigstens teilweise innerhalb des Gehäuses angeordnet. Das Projektil bzw. ein das Projektil führender Führungskanal kann zumindest teilweise koaxial innerhalb eines von dem Ultraschallhorn eingeschlossenen Volumen angeordnet sein. Der Führungskanal bildet insbesondere ein Lager, vorzugsweise ein Linearlager, für das Projektil aus, wobei es sich bei dem Lager insbesondere um ein Gleitlager handeln kann. Vorzugsweise weist der Führungskanal eine Antihaft-, Gleit, und/oder Korrosionsbeschichtung an Flächen auf, welche in einem Betriebszustand an weiteren Flächen des Projektils reiben, wie beispielsweise eine Mantelfläche des Führungskanals. Bei der Antihaft-, Gleit, und/oder Korrosionsbeschichtung kann es sich beispielsweise um eine PTFE-, Keramik-, Kohlenstoff-Beschichtung oder dergleichen handeln. Der Führungskanal ist insbesondere als ein länglicher Kanal ausgebildet.

Zu einer Beschleunigung des Projektils kann ein Antriebseinrichtung für das Projektil vorgesehen sein. Bei der Antriebseinrichtung handelt es sich beispielsweise um eine elektromagnetische, eine mechanische, eine hydraulische und/oder eine pneumatische Antriebseinrichtung. Die Antriebseinrichtung könnte Teil der Lithotripsievorrichtung sein, insbesondere wenn es sich beispielsweise um eine elektromagnetische Antriebseinrichtung handelt. Dazu kann die Antriebseinrichtung zumindest teilweise, vorzugsweise zumindest zu einem Großteil, innerhalb des Gehäuses angeordnet sein. Alternativ kann die Antriebseinrichtung von der Lithotripsievorrichtung separat ausgebildet sein. Beispielsweise kann die Antriebseinrichtung Teil eines Lithotripsiesystems sein, welches ebenfalls einen die Lithotripsievorrichtung umfassenden Lithotripter aufweist. Auch könnte die Antriebseinrichtung in ein Steuergerät eines solchen Lithotripsiesystems integriert sein. Die Antriebseinrichtung kann in etwa ein Linear-Aktor bzw. Motor, insbesondere linearer Elektromotor sein. Alternativ kann die Antriebseinrichtung ein Pneumatik-Aktor sein. Der Pneumatik-Aktor weist wenigstens einen Druckgenerator auf, welcher mit dem Führungskanal der Lithotripsievorrichtung verbunden ist. Um mittels des Druckgenerators das Projektil zu beschleunigen kann dann ein von dem Druckgenerator aufgebauter Druck schlagartig auf den Führungskanal gegeben werden, wodurch das Projektil beschleunigt werden kann.

Vorteilhaft kann eine Kühlung weiter verbessert werden, wenn die Kappe die Sonotrode und/oder das Ultraschallhorn koaxial umgebend angeordnet ist, da eine homogenere Wärmeübertragung auf eine große Fläche der Sonotrode und/oder des Ultraschallhorns erzielt werden kann. Insbesondere ist die Kappe derart angeordnet, dass eine Drehsymmetrieachse und/oder eine Mittelachse der Kappe mit einer Drehsymmetrieachse und/oder eine Mittelachse der Sonotrode und/oder des Ultraschallhorns identisch ist. Die Drehsymmetrieachse ist dabei vorzugsweise identisch mit einer Mittelachse des Irrigationskanals.

Ferner wird vorgeschlagen, dass die Kappe korrespondierend zu der Sonotrode und/oder des Ultraschallhorns ausgebildet ist. Es kann vorteilhaft eine Bauweise weiter verbessert werden, so dass eine kompakte, effiziente Bauraumgestaltung erzielt, aber gleichzeitig noch eine ausreichende Größe des Abführungskanals und somit ein ausreichender Flüssigkeitstransport sichergestellt werden kann. Insbesondere sind die den Raum begrenzenden und einander gegenüberliegenden Wandungen der Kappe und der Sonotrode und/oder des Ultraschallhorns zueinander korrespondierend ausgebildet. Ist beispielsweise eine Wandung der Sonotrode konkav, so ist die gegenüberliegende Wandung der Kappe ebenfalls konkav. Unter "korrespondierend" soll insbesondere in Beziehung stehend, wie beispielsweise Puzzleteile zueinander korrespondierend ausgebildet seien können, um miteinander verbindbar zu sein. Ferner soll unter korrespondierend auch übereinstimmend verstanden werden.

Um vorteilhaft eine Montage, einen Austausch und/oder eine Reinigung der Kappe zu vereinfachen, wird vorgeschlagen, dass die Kappe wenigstens eine axiale Öffnung aufweist, durch welche sich die Sonotrode hindurch erstreckt. Dadurch kann die Kappe vorteilhaft einfach auf die Sonotrode aufgeschoben bzw. herabgezogen werden. Die axiale Öffnung weist insbesondere einen Innendurchmesser auf, welcher einem Außendurchmesser der Sonotrode zumindest im Wesentlichen entspricht. Bei der axialen Öffnung handelt es sich insbesondere um eine distale Öffnung der Kappe. Die axiale Öffnung ist insbesondere an einer Position innerhalb eines Bereichs, wobei der Bereich entlang der Haupterstreckungsrichtung der Sonotrode gemessen eine Länge von höchstens einem Viertel, vorzugsweise höchstens einem Achtel und besonders bevorzugt höchstens einem Sechzehntel einer Wellenlänge des Ultraschalls entspricht, um einen Schwingungsknoten der Ultraschallwellen angeordnet oder am Ort eines Schwingungsknoten der Ultraschallwellen selbst angeordnet.

Um vorteilhaft eine Montage, einen Austausch und/oder eine Reinigung der Kappe zu vereinfachen, wird vorgeschlagen, dass die Kappe wenigstens eine weitere axiale Öffnung aufweist, durch welche sich das Ultraschallhorn hindurch erstreckt. Ferner erstreckt sich insbesondere das Gehäuse zumindest teilweise durch die weitere axiale Öffnung. Die weitere axiale Öffnung weist insbesondere einen Innendurchmesser auf, welcher einem Außendurchmesser des Ultraschallhorns und/oder des Gehäuses entspricht. Die weitere axiale Öffnung ist insbesondere an einer Position innerhalb eines Bereichs, wobei der Bereich entlang der Haupterstreckungsrichtung der Sonotrode gemessen eine Länge von höchstens einem Viertel, vorzugsweise höchstens einem Achtel und besonders bevorzugt höchstens einem Sechzehntel einer Wellenlänge des Ultraschalls entspricht, um einen Schwingungsknoten der Ultraschallwellen angeordnet oder am Ort eines Schwingungsknoten der Ultraschallwellen selbst angeordnet. Die Öffnung ist insbesondere eine proximale Öffnung der Kappe. Die weitere axiale Öffnung liegt insbesondere der axialen Öffnung gegenüber. Die weitere axiale Öffnung ist insbesondere an einer Position innerhalb eines Bereichs, wobei der Bereich entlang der Haupterstreckungsrichtung der Sonotrode gemessen eine Länge von höchstens einem Viertel, vorzugsweise höchstens einem Achtel und besonders bevorzugt höchstens einem Sechzehntel einer Wellenlänge des Ultraschalls entspricht, um einen Schwingungsknoten der Ultraschallwellen angeordnet oder am Ort eines Schwingungsknoten der Ultraschallwellen selbst angeordnet

Weiter wird vorgeschlagen, dass die Kappe zu einer zumindest teilweise die Sonotrode und/oder das Ultraschallhorn umgebenden Anordnung wenigstens einen Anschlag aufweist. Es kann vorteilhaft eine kompakte Anordnung der Kappe erzielt werden, wobei insbesondere ein Halt der Kappe verbessert werden kann und gleichzeitig vermieden werden kann, dass sich die Kappe durch Ultraschallwellen beschädigt, undicht wird oder sich ablöst. Der Anschlag ist insbesondere einstückig mit einer den Raum zwischen Kappe und Ultraschallhorn und/oder Sonotrode begrenzenden Wandung der Kappe ausgebildet. Der Anschlag ist vorzugsweise als eine Stufe ausgebildet, welche sich ringförmig entlang eines gesamten Umfangs dieser Wandung erstreckt. Die Kappe liegt proximalseitig insbesondere an dem Ultraschallhorn und/oder dem Gehäuse an. Das Ultraschallhorn und/oder das Gehäuse kann einen zu dem Anschlag korrespondieren ausgebildeten korrespondierenden Anschlag aufweisen, an welchem der Anschlag in einem montierten Zustand anliegt. Der korrespondierende Anschlag ist insbesondere einstückig mit einer den Raum zwischen Kappe und Ultraschallhorn und/oder Sonotrode begrenzenden Wandung der Ultraschallhorns und/oder einer Gehäusewand des Gehäuses ausgebildet. Der Anschlag und/oder der korrespondierende Anschlag ist besonders bevorzugt an einer Position innerhalb eines Bereichs, wobei der Bereich entlang der Haupterstreckungsrichtung der Sonotrode gemessen eine Länge von höchstens einem Viertel, vorzugsweise höchstens einem Achtel und besonders bevorzugt höchstens einem Sechzehntel einer Wellenlänge des Ultraschalls entspricht, um einen Schwingungsknoten der Ultraschallwellen angeordnet oder am Ort eines Schwingungsknoten der Ultraschallwellen selbst angeordnet. Weiter vorteilhaft kann eine Übertragung von Ultraschallwellen auf die Kappe vermieden werden, wodurch ein Energieverlust bei der Übertragung von Ultraschallwellen oder Stößen verringert werden kann.

Es wird weiterhin vorgeschlagen, dass die Lithotripsievorrichtung wenigstens eine Dichtung, welche einen den Abführungskanal wenigstens teilweise ausbildenden Spalt zwischen der die Sonotrode und/oder das Ultraschallhorn zumindest teilweise umgebend angeordnet Kappe gegenüber einer Umgebung fluiddicht abdichtet. Es kann vorteilhaft eine kompakte Anordnung der Kappe erzielt werden, wobei insbesondere ein Halt der Kappe verbessert und gleichzeitig vermieden werden kann, dass der Abführungskanal undicht wird. Die Dichtung ist insbesondere als ein Dichtring und/oder eine Dichthülse ausgebildet. Ferner ist denkbar, dass die Dichtung einstückig mit der Kappe verbunden ist. Die Dichtung kann insbesondere im proximalen Abschnitt der Kappe angeordnet sein. Die Dichtung kann beispielsweise in die Wandung des Gehäuses der Kappe, des Gehäuses und/oder des Ultraschallhorns eingelassen sein. Die Dichtung ist besonders bevorzugt an einer Position innerhalb eines Bereichs, wobei der Bereich entlang der Haupterstreckungsrichtung der Sonotrode gemessen eine Länge von höchstens einem Viertel, vorzugsweise höchstens einem Achtel und besonders bevorzugt höchstens einem Sechzehntel einer Wellenlänge des Ultraschalls entspricht, um einen Schwingungsknoten der Ultraschallwellen angeordnet oder am Ort eines Schwingungsknoten der Ultraschallwellen selbst angeordnet. Auch kann eine Dichtung am distalen Abschnitt angeordnet sein. Weiter vorteilhaft kann eine Übertragung von Ultraschallwellen auf die Kappe vermieden werden, wodurch ein Energieverlust bei der Übertragung von Ultraschallwellen oder Stößen verringert werden kann.

Es wird ferner vorgeschlagen, dass die Kappe wenigste einen mit dem Abführungskanal fluidtechnisch verbundenen Anschluss aufweist. Es kann eine besonders kompakte Bauweise erzielt werden, da das Irrigat aus dem Abführungskanal außerhalb der Lithotripsievorrichtung abfließen kann, wodurch ein Durchleiten des Irrigats durch die Lithotripsievorrichtung und insbesondere durch Komponenten der Lithotripsievorrichtung, welche der Sonotrode und/oder dem Ultraschallhorn nachgeschaltet sind, wie beispielsweise dem Führungskanal, dem Ultraschallgenerator und/oder einem Antrieb für das Projektil. Auch kann vermieden werden, dass elektrische Komponenten der Lithotripsievorrichtung wie beispielsweise der Ultraschallgenerator oder dergleichen, in Kontakt zu dem Irrigat kommen, wodurch eine Sicherheit der Lithotripsievorrichtung verbessert werden kann. Die den Abführungskanal begrenzende Wandung der Kappe weist insbesondere wenigstens eine Ausnehmung auf, welche als Öffnung zum Anschluss hin ausbildet. Der Anschluss ist insbesondere wiederum mit einer Förderreinrichtung, wie beispielsweise einer Pumpe, verbunden, welche insbesondere dazu eingerichtet ist, das Irrigat zu fördern. Ferner ist denkbar, dass die Kappe wenigstens zwei oder mehrere solcher Anschlüsse aufweist, welche längs der Kappe und/oder entlang eines Umfangs der Kappe versetzt zueinander angeordnet seien könnten. Die den Abführungskanal begrenzende Wandung der Kappe kann insbesondere wenigstens zwei oder mehrere solcher Ausnehmungen aufweisen, welche längs der Kappe und/oder entlang eines Umfangs der Kappe versetzt zueinander angeordnet sein könnten.

Die Kappe ist insbesondere mit weiteren Komponenten der Lithotripsievorrichtung fest verbunden und/oder verbindbar, wie beispielsweise der Sonotrode, dem Ultraschallhorn und/oder einem Gehäuse der Lithotripsievorrichtung. Ist die Kappe derart verbindbar, so kann eine Reinigbarkeit verbessert werden, da die Kappe dann einzeln entfernt und gereinigt werden kann. Weiter vorteilhaft kann derart der Abführungskanal zerlegt und gereinigt werden. Um eine einfache Reinigung zu erzielen und eine damit in Verbindung stehende Demontage bzw. Montage zu erleichtern, wird insbesondere vorgeschlagen, dass die Lithotripsievorrichtung wenigstens einen zu einer zumindest teilweise die Sonotrode und/oder das Ultraschallhorn umgebenden Anordnung der Kappe eingerichteten Schnellverbinder, wie beispielsweise ein Bajonettverbinder, ein Schnellspannverbinder, ein Schraubverbinder oder dergleichen, welcher wenigstens teilweise von der Kappe ausgebildet ist. Der Schnellverbinder ist insbesondere dazu eingerichtet, eine werkzeuglose und zerstörungsfrei lösbare Verbindung herzustellen, wie insbesondere eine kraft- und/oder formschlüssige Verbindung. Der Schnellverbinder ist insbesondere dazu eingerichtet, die Kappe mit dem Gehäuse zu verbinden. Die Kappe weist insbesondere wenigstens ein Verbindungsteil auf, welcher wenigstens teilweise den Schnellverbinder ausbildet. Denkbar ist, dass die Kappe wenigstens zwei oder mehrere Verbindungsteile aufweist, welche insbesondere entlang eines Umfangs der Kappe versetzt zueinander angeordnet seien können. Bei dem Verbindungsteil kann es sich beispielsweise um eine Bajonettverbinderführung handeln, welche sich insbesondere aus einem Längsschlitz und einem an dessen Ende rechtwinklig ansetzenden Querschlitz zusammensetzt. Alternativ kann es sich bei dem Verbindungsteil auch um eine Rastlasche bzw. -haken, ein Gewinde oder dergleichen handeln. Insbesondere bildet das Gehäuse zumindest teilweise den Schnellverbinder aus. Das Gehäuse weist vorzugsweise ein zu dem Verbindungsteil korrespondierend ausgebildetes korrespondierendes Verbindungsteil auf, welches zumindest teilweise den Schnellverbinder ausbildet. Denkbar ist, dass das Gehäuse wenigstens zwei oder mehrere korrespondierende Verbindungsteile aufweist, welche insbesondere entlang eines Umfangs des Gehäuses versetzt zueinander angeordnet seien können. Bei dem korrespondierenden Verbindungsteil kann es sich beispielsweise um einen Bajonettverbinderkopf handeln, welcher insbesondere in den Querschlitz des Verbindungselements einführbar und durch Verdrehen im Längsschlitz eine feste Verbindung bewirkt. Alternativ kann es sich bei dem korrespondierenden Verbindungsteil auch um eine Rastnase, ein Gegengewinde oder dergleichen handeln. Auch ist denkbar, dass die Kappe anstelle des Gehäuses wenigstens ein korrespondierendes Verbindungsteil aufweist und das Gehäuse anstelle der Kappe das Verbindungsteil aufweist. Für den Fall, dass mehrere Verbindungsteile und korrespondierende Verbindungsteile vorhanden sind, ist denkbar, dass die Kappe sowohl Verbindungsteile als auch korrespondierende Verbindungsteile aufweist und das Gehäuse mehrere korrespondierende Verbindungsteile und Verbindungsteile aufweist.

Ferner wird vorgeschlagen, dass die Kappe zumindest teilweise, vorzugsweise zumindest zu einem Großteil und besonders bevorzugt vollständig, transparent ausgebildet ist. Es kann vorteilhaft durch die Kappe der Abführungskanal und das durch den Abführungskanal geförderte Irrigat sichtbar gemacht werden, wodurch auch von dem Irrigat abtransportierte Bruchstücke von Konkrementen erkennbar werden, so dass in einem Betrieb eine Funktionsweise der Lithotripsievorrichtung überprüft werden kann. Weiter vorteilhaft kann leicht festgestellt werden, ob der Abführungskanal evtl. blockiert oder verstopft ist. Alternativ oder zusätzlich könnte die Kappe mit einem Indikator, wie beispielsweise einem PH-Indikator, versehen sein, welcher die Farbe der Kappe wechselt, sobald ein Irrigat durch die Kappe transportiert wird.

Bei der Kappe kann es sich um eine Kappe handeln, welche als ein Mehrwegbauteil ausgebildet ist. Die Kappe ist insbesondere aus einem einen Autoklavierprozess widerstehenden Kunststoff ausgebildet, wie beispielsweise PEEK, PPS, PVDF, PEO, PPSU, PSU oder ein Gemisch dieser. Um vorteilhaft eine Hygiene der Lithotripsievorrichtung zu verbessern, um beispielsweise Verunreinigungen durch fehlerhafte Anwendung eines Autoklavierprozesses zu vermeiden, wird vorgeschlagen, dass die Kappe als ein Einwegbauteil eingerichtet ist. Unter einem "Einwegbauteil" soll insbesondere ein Bauteil verstanden werden, welches nur einmalig verwendbar ist und vorzugweise nicht zu einem Autoklavierprozess eingerichtet ist. Dies ist beispielsweise der Fall, wenn die Kappe wenigstens teilweise aus einem Kunststoff gebildet ist, welcher bei Temperaturen eines einem Fachmann bekannten Autoklavierprozesses sich vorzugsweise gezielt verformt oder beschädigt wird. Die Kappe ist insbesondere aus einem einen Autoklavierprozess nicht widerstehenden Kunststoff ausgebildet, wie beispielsweise POM-C, PETP oder ein Gemisch dieser.

Außerdem wird vorgeschlagen, dass der Abführungskanal die Sonotrode und/oder das Ultraschallhorn zumindest teilweise, vorzugsweise zumindest zu einem Großteil und besonders bevorzugt vollständig entlang ihres/deren Umfangs umgibt. Es kann vorteilhaft eine Kühlung weiter verbessert werden, da derart die Sonotrode und/oder das Ultraschallhorn großflächig von dem zur Kühlung genutzten Irrigat umspült werden können.

Ferner wird vorgeschlagen, dass der Abführungskanal wenigstens abschnittsweise trichterförmig ausgebildet ist. Es kann vorteilhaft eine Kühlung besonders verbessert werden, da derart die Sonotrode und/oder das Ultraschallhorn besonders großflächig von dem zur Kühlung genutzten Irrigat umspült werden können. Ferner kann der Abführungskanal wenigstens teilweise röhrenförmig ausgebildet sein.

Weiter wird vorgeschlagen, dass der Irrigationskanals eine Haupterstreckung aufweist und die Sonotrode und das Ultraschallhorn eine Gesamthaupterstreckung aufweisen, wobei die Haupterstreckung des Irrigationskanals kleiner ist als die Gesamthaupterstreckung der Sonotrode und des Ultraschallhorns. Es kann vorteilhaft eine Konstruktion der Lithotripsievorrichtung vereinfacht werden, da vermieden werden kann, den Irrigationskanal vollständig durch die gesamte Sonotrode und/oder Ultraschallhorn zu führen. Insbesondere ist die Haupterstreckung des Irrigationskanals kleiner als die Haupterstreckung der Sonotrode. Unter einer "Haupterstreckung" eines Objekts soll insbesondere eine Erstreckung des Objekts entlang dessen Haupterstreckungsrichtung verstanden werden.

Es wird zudem vorgeschlagen, dass die Sonotrode und/oder das Ultraschallhorn zu einer fluidtechnischen Verbindung des Irrigationskanal mit dem Abführungskanal wenigstens eine radiale Öffnung aufweist. Besonders bevorzugt ist die radiale Öffnung an einer Position innerhalb eines Bereichs, wobei der Bereich entlang der Haupterstreckungsrichtung der Sonotrode gemessen eine Länge von höchstens einem Viertel, vorzugsweise höchstens einem Achtel und besonders bevorzugt höchstens einem Sechzehntel einer Wellenlänge des Ultraschalls entspricht, um einen Schwingungsbauchs der Ultraschallwellen angeordnet oder am Ort eines Schwingungsbauchs der Ultraschallwellen selbst angeordnet. Es kann vorteilhaft auf einfache Art und Weise eine Verbindung zwischen dem Irrigationskanal und dem Abführungskanal geschaffen werden, wobei insbesondere eine Beschädigung der Sonotrode durch einen durch die Öffnung geschaffenen Abführungskanal undicht wird. Die radiale Öffnung ist insbesondere an einem proximalen Endabschnitt der Sonotrode und/oder des Ultraschallhorns angeordnet. Die radiale Öffnung ist beispielsweise in einer den Abführungskanal begrenzenden Wandung der Sonotrode und/oder des Ultraschallhorns angeordnet. Ferner ist denkbar, dass die Sonotrode wenigstens zwei oder mehrere solcher radialen Öffnungen umfasst, welche längs und/oder entlang eines Umfangs der Sonotrode und/oder des Ultraschallhorns versetzt zueinander angeordnet sein könnten. Ferner kann die Sonotrode zusätzliche radiale Öffnungen aufweisen, welche an einem distalen Endabschnitt der Sonotrode angeordnet sind, um beispielsweise ein Irrigat in den Irrigationskanal hinein zu fördern. Unter einem "Endabschnitt" eines Objekts soll insbesondere ein Abschnitt verstanden werden, welcher sich von einem Ende des Objekts in Richtung des Objekts um höchstens 10%, vorzugsweise höchstens 5% und besonders bevorzug um höchstens 1% einer Haupterstreckung des Objekts in Richtung einer Mitte des Objekts hin erstreckt.

Um eine einfache und fluiddichte Bauform zu erzielen wird insbesondere vorgeschlagen, dass die Kappe die radiale Öffnung der Sonotrode und/oder des Ultraschallhorns überdeckend angeordnet ist. Derart schließt die Kappe sowohl die radiale Öffnung als auch den Abführungskanal ein, wodurch ein fluiddichtes und insbesondere austauschbares Modul geschaffen werden kann. Außerdem wird vorgeschlagen, dass der Irrigationskanal innerhalb der Sonotrode und/oder des Ultraschallhorns wenigstens eine Abwinklung aufweist, welche sich zur radialen Öffnung der Sonotrode und/oder des Ultraschallhorns hin erstreckt. Es kann vorteilhaft eine besonders gezielte Kühlung und/oder Abführung des Irrigats erzielt werden. Unter einer "Abwinklung" soll insbesondere ein Kanalabschnitt des Irrigationskanals verstanden werden, welcher wenigstens zwei weitere Kanalabschnitte des Irrigationskanal winklig miteinander verbindet. Unter "winklig" soll insbesondere von zumindest im Wesentlichen parallel verschieden verstanden werden. Vorzugsweise entspricht ein Winkel zwischen den beiden über die Abwinklung verbundenen Kanalabschnitten mindestens 0° und höchstens 90°.

Ferner wird ein Lithotripter mit wenigstens einer solchen Lithotripsievorrichtung beansprucht.

Hierdurch kann vorteilhaft eine Kühlung verbessert werden. Weiter vorteilhaft kann eine Bauweise der Lithotripsievorrichtung vereinfacht werden.

Außerdem wird eine Kappe wenigstens einer solchen Lithotripsievorrichtung offenbart.

Hierdurch kann vorteilhaft eine Kühlung verbessert werden. Weiter vorteilhaft kann eine Bauweise der Lithotripsievorrichtung vereinfacht werden.

Zusätzlich wird ein Bausatz für einen Lithotripter mit wenigstens einer solchen Lithotripsievorrichtung sowie mit wenigstens einer weiteren Kappe beansprucht. Hierdurch kann vorteilhaft eine Kühlung verbessert werden. Weiter vorteilhaft kann eine Bauweise der Lithotripsievorrichtung vereinfacht werden. Ferner kann eine Hygiene und eine Reinigung verbessert werden. Insbesondere kann ein solcher Bausatz wenigstens zwei oder mehrere solche weiterer Kappen umfassen. Die Kappen könnten dabei zumindest teilweise voneinander unterschiedlich ausgebildet sein. Beispielsweise könnten sich die Kappen in einem montierten Zustand durch ein von den Kappen und der Sonotrode und/oder dem Ultraschallhorn eingeschlossenen Spalt voneinander unterscheiden, so dass vorteilhaft je nach Kombination der genutzten Kappe unterschiedlich große Abführungskanäle realisierbar sind. Ferner ist denkbar, dass die Kappen, welche vorzugsweise unterschiedlich voneinander ausgebildet sind sich durch eine Kodierung, wie beispielsweise eine Farbkodierung, einen RFID-Chip-Kodierung, oder dergleichen voneinander unterscheiden.

Zudem wird ein Lithotripsiesystem mit wenigstens einem solchen Lithotripter und wenigstens einem Steuergerät, welches zu einer Steuerung des Lithotripters eingerichtet ist, beansprucht. Hierdurch kann vorteilhaft eine Kühlung verbessert werden. Weiter vorteilhaft kann eine Bauweise des Lithotripsiesystems vereinfacht werden Das Steuergerät umfasst insbesondere wenigstens einen Prozessor, welcher zur Ausführung eines Programms zur Ansteuerung des Lithotripters eingerichtet ist. Bei dem Programm kann es sich beispielsweise um ein Programm zur Ausführung eines Verfahrens zum Betrieb der Lithotripsievorrichtung handeln. Ferner kann das Steuergerät wenigstens einen Speicher aufweisen, auf welchem wenigstens das zur Ausführung im Prozessor eingerichtete Programm hinterlegt sein kann.

Weiter wird ein Verfahren zum Betrieb und/oder zur Herstellung wenigstens einer solchen Lithotripsievorrichtung offenbart. Hierdurch kann vorteilhaft eine Kühlung der Lithotripsievorrichtung verbessert werden. Weiter vorteilhaft kann eine Montage bzw. Demontage der Lithotripsievorrichtung vereinfacht werden Bei dem Verfahren kann es sich beispielsweise um ein Testverfahren handeln, welches insbesondere zu einem extrakorporalen Testbetrieb der Lithotripsievorrichtung eingerichtet ist. Der extrakorporale Testbetrieb kann dabei von einem chirurgischen oder therapeutischen Betrieb der Lithotripsievorrichtung verschieden sein, da dabei nicht in etwa innerhalb eines Körpers Konkremente zu Testzwecken zerstört werden können, um beispielsweise eine Anwendungsdauer der Lithotripsievorrichtung zu testen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung eines Lithotripsiesystems mit einer einen Lithotripter ausbildenden Lithotripsievorrichtung in einer perspektivischen Ansicht,
- Fig. 2: eine schematische Darstellung eines Teils der Lithotripsievorrichtung in einer Schnittansicht,
- Fig. 3: eine schematische Darstellung eines Teils der Lithotripsievorrichtung mit einer Kappe in einer Schnittansicht,
- Fig. 4: einen schematischen Ablaufplan eines beispielhaften Verfahrens zur Herstellung einer Lithotripsievorrichtung,
- Fig. 5: eine schematische Darstellung eines Bausatzes für einen Lithotripter mit einer Lithotripsievorrichtung und wenigstens einer zusätzlichen Kappe,
- Fig. 6: einen schematischen Ablaufplan eines beispielhaften Verfahrens zum Betrieb einer Lithotripsievorrichtung und
- Fig. 7: eine schematische Darstellung einer weiteren Lithotripsievorrichtung in einer Schnittansicht.

### Beschreibung der Ausführungsbeispiele

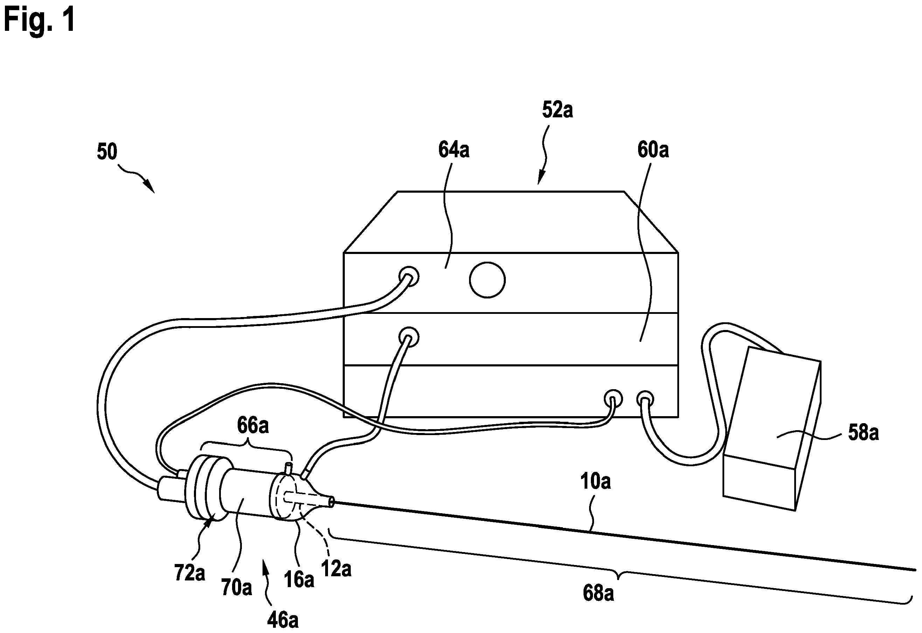

Fig. 1 zeigt eine schematische Darstellung eines Lithotripsiesystems 50a in einer perspektivischen Ansicht. Das Lithotripsiesystem 50a weist wenigstens einen Lithotripter 46a auf. Der Lithotripter 46a ist im vorliegenden Fall als ein intrakorporaler Lithotripter 46a ausgebildet. Alternativ könnte ein Lithotripter auch als ein extrakorporaler Lithotripter ausgebildet sein.

Der Lithotripter 46a weist wenigstens eine Lithotripsievorrichtung auf. Im vorliegenden Fall bildet die Lithotripsievorrichtung den Lithotripter 46a vollständig aus. Alternativ könnte die Lithotripsievorrichtung nur einen Teil eines Lithotripters ausbilden.

Das Lithotripsiesystem 50a weist wenigstens ein Steuergerät 52a auf. Das Steuergerät 52a ist zumindest zu einer Steuerung der Lithotripsievorrichtung eingerichtet. Die Lithotripsievorrichtung ist mit dem Steuergerät 52a verbunden. Das Steuergerät 52a besitzt zumindest eine Steuerelektronik (nicht dargestellt). Zu einer Ansteuerung der Lithotripsievorrichtung ist die Steuerelektronik elektrisch und/oder elektronisch mit dieser verbunden. Die Steuerelektronik enthält zumindest einen Prozessor. Ferner enthält die Steuerelektronik wenigstens einen Speicher. Auf dem Speicher ist ein Betriebsprogramm hinterlegt. Das Betriebsprogramm ist von dem Prozessor ausführbar.

Das Lithotripsiesystem 50a weist wenigstens ein Betätigungsmittel 58a auf. Das Betätigungsmittel 58a ist zu einer Aktivierung und/oder Deaktivierung der Lithotripsievorrichtung ausgebildet. Das Betätigungsmittel 58a ist mit dem Steuergerät 52a und zwar insbesondere mit der Steuerelektronik des Steuergeräts 52a verbunden. Im vorliegenden Fall ist das Betätigungsmittel 58a als ein Fußschalter ausgebildet. Alternativ könnte ein Betätigungsmittel auch als ein anderer elektrischer und/oder elektronischer Schalter ausgebildet sein, wie beispielsweise als ein Druckknopf, einen Kippschalter oder dergleichen, welcher insbesondere Teil einer Lithotripsievorrichtung seien könnte. Auch könnte eine Bedienung der Lithotripsievorrichtung über ein externes Gerät, insbesondere ein Handgerät, wie beispielsweise ein Tablet, ein Smartphone, eine Smartwatch oder dergleichen erfolgen.

Das Lithotripsiesystem 50a weist ferner eine Fördereinrichtung 60a auf. Die Fördereinrichtung 60a ist im vorliegenden Fall als eine Pumpe ausgebildet. Die Fördereinrichtung 60a ist zur Förderung eines Irrigats eingerichtet. Dazu ist die Fördereinrichtung 60a fluidtechnisch mit der Lithotripsievorrichtung verbunden. Die Fördereinrichtung 60a dient zum Absaugen eines Irrigats und/oder zu einem Freispulen mittels eines Irrigats bei einem Lithotripsievorgang.

Bei dem Irrigat kann es sich um eine Flüssigkeit handeln, wie in etwa eine Kochsalzlösung. Beispielsweise können mit dem Irrigat Bruchteile eines bei einem Lithotripsievorgang zerstörten Konkrements abtransportiert werden.

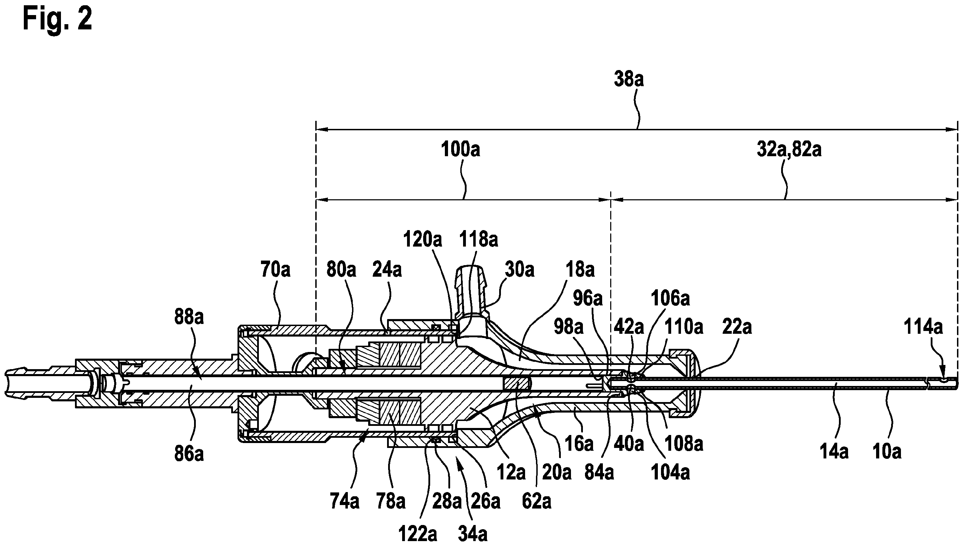

Zur Erzeugung von Stoßimpulsen, wie beispielsweise mittels eines Projektils 62a, der Lithotripsievorrichtung ist wenigstens eine Antriebseinrichtung 64a vorgesehen (vgl. Fig. 2). Die Antriebseinrichtung 64a ist im vorliegenden Fall separat von der Lithotripsievorrichtung ausgebildet. Die Antriebseinrichtung 64a ist Teil des Lithotripsiesystems 50a. Die Antriebseinrichtung 64a ist Teil des Steuergeräts 52a. Die Antriebseinrichtung 64a ist im vorliegenden Fall als eine pneumatische Antriebseinrichtung 64a ausgebildet. Die Antriebseinrichtung 64a ist als ein Druckluftgenerator ausgebildet. Alternativ könnte es sich bei der Antriebseinrichtung um eine mechanische, elektromagnetische oder eine Hydraulische Antriebseinrichtung handeln. Alternativ könnte eine solche Antriebseinrichtung auch Teil einer solchen Lithotripsievorrichtung sein, beispielsweise, wenn diese Antriebseinrichtung als eine elektromagnetische Antriebseinheit, wie in etwa ein linearer Aktor. Auch könnte eine Antriebeinrichtung ein von einem Steuergerät separates Antriebsgerät ausbilden.

Die Lithotripsievorrichtung besitzt einen proximalen Abschnitt 66a. Der proximale Abschnitt 66a ist bei einer Bedienung einem Bediener der Lithotripsievorrichtung zugewandt. Der proximale Abschnitt 66a ist bei einer Bedienung einem Patienten abgewandt. Der proximale Abschnitt 66a liegt bei einer Behandlung eines Patienten außerhalb des Patienten.

Ferner besitzt die Lithotripsievorrichtung einen distalen Abschnitt 68a. Der distale Abschnitt 68a ist bei einer Bedienung einem Bediener der Lithotripsievorrichtung abgewandt. Der distale Abschnitt 68a ist bei einer Bedienung einem Patienten zugewandt. Der distale Abschnitt 68a liegt bei einer Behandlung eines Patienten zumindest teilweise innerhalb des Patienten.

Die Lithotripsievorrichtung weist ein Gehäuse 70a auf. Das Gehäuse 70a ist hohlzylinderförmig.

Das Gehäuse 70a bildet zumindest teilweise den proximalen Abschnitt 66a der Lithotripsievorrichtung aus. Das Gehäuse 70a dient zur Anordnung weiterer Komponenten der Lithotripsievorrichtung.

Ferner weist die Lithotripsievorrichtung eine Handhabe 72a auf. Die Handhabe 72a ist zu einer manuellen Bedienung der Lithotripsievorrichtung ausgebildet. Die Handhabe 72a ist von dem Gehäuse 70a ausgebildet. Die Handhabe 72a bildet zumindest teilweise den proximalen Abschnitt 66a aus. Alternativ könnte insbesondere bei einer robotischen Ansteuerung einer solchen Lithotripsievorrichtung auch auf eine Handhabe 72a verzichtet werden oder zu einer Anbindung an einen Roboterarm genutzt werden.

Fig. 2 zeigt eine schematische Darstellung eines Teils der Lithotripsievorrichtung in einer Schnittansicht.

Die Lithotripsievorrichtung weist wenigstens einen Ultraschallgenerator 74a auf. Der Ultraschallgenerator 74a ist dazu ausgebildet, Ultraschallwellen bereitzustellen. Zu einer Ansteuerung ist der Ultraschallgenerator 74a mit dem Steuergerät 52 und zwar insbesondere der Steuerelektronik verbunden. Der Ultraschallgenerator 74a ist zumindest teilweise in dem Gehäuse 70a angeordnet. Im vorliegenden Fall ist der Ultraschallgenerator 74a vollständig in dem Gehäuse 70a angeordnet. Der Ultraschallgenerator 74a bildet zumindest teilweise den proximalen Abschnitt 66a der Lithotripsievorrichtung aus.

Der Ultraschallgenerator 74a enthält wenigstens einen Ultraschallaktor c. Im vorliegenden Fall weist der Ultraschallgenerator 74a mehrere Ultraschallaktoren 78a auf. Der Übersichtlichkeit halber sind etwaige Anschluss- und/oder Kontaktierungsmittel und/oder Isolationen der Ultraschallaktoren 78a nicht in der Fig. 2 dargestellt.

Der Ultraschallgenerator 74a weist zwei Ultraschallaktoren 78a auf. Die Ultraschallaktoren 78a sind aneinander anliegend gestapelt angeordnet. Der Übersichtlichkeit halber ist in den Zeichnungen nur ein Ultraschallaktor 78a mit einem Bezugszeichen versehen. Die Ultraschallaktoren 78a sind zumindest im Wesentlichen identisch zueinander ausgebildet. Im Folgenden ist nur ein Ultraschallaktor 78a näher beschrieben. Diese Beschreibung ist auch auf die weiteren Ultraschallaktoren 78a übertragbar. Im vorliegenden Fall ist der Ultraschallaktor 78a als ein Piezoaktor ausgebildet. Der Ultraschallaktor 78a ist zylinderplattenförmig ausgebildet.

Ferner weist die Lithotripsievorrichtung wenigstens ein Ultraschallhorn 12a auf. Das Ultraschallhorn 12a ist zur Übertragung von Ultraschallwellen ausgebildet. Das Ultraschallhorn 12a liegt an dem Ultraschallaktor 78a an. Das Ultraschallhorn 12a ist ferner zur Fokussierung von Ultraschallwellen ausgebildet.

Das Ultraschallhorn 12a ist zumindest teilweise in dem Gehäuse 70a angeordnet. Das Ultraschallhorn 12a bildet zumindest teilweise den proximalen Abschnitt 66a der Lithotripsievorrichtung aus. Das Ultraschallhorn 12a ist zumindest teilweise außerhalb des Gehäuse 70a angeordnet. Das Ultraschallhorn 12a weist die Form eines Rotationskörpers auf. Im vorliegenden Fall weist das Ultraschallhorn 12a eine hohlzylinderartige Form auf. Das Ultraschallhorn 12a ist zumindest teilweise aus Metall, insbesondere Stahl oder Titan ausgebildet. Der Ultraschallgenerator 74a liegt an dem Ultraschallhorn 12a an.

Das Ultraschallhorn 12a weist eine Verjüngung auf. Die Verjüngung liegt außerhalb des Gehäuses 70a. Die Verjüngung ist frei von Stufen. Die Verjüngung weist einen stetigen Verlauf auf. Der Verlauf der Verjüngung ist konkav. Der Verlauf der Verjüngung ist exponentiell. Alternativ oder zusätzlich könnte die Verjüngung auch stufenförmig oder katenoidalförmig sein. Das Ultraschallhorn 12a weist eine Ausnehmung 80a auf. Die Ausnehmung 80a verläuft koaxial entlang einer Rotationsachse des Ultraschallhorns 12a.

Die Lithotripsievorrichtung umfasst wenigstens ein Projektil 62a. Das Projektil 62a ist linear beweglich gelagert. Das Projektil 62a ist im Bereich des Ultraschallhorns 12a beweglich gelagert angeordnet. In wenigstens einem Betriebszustand ist das Projektil 62a dazu ausgebildet, die Sonotrode 10a und/oder das Ultraschallhorn 12a zumindest mittelbar zu beaufschlagen. Im vorliegenden Fall beaufschlagt das Projektil 62a die Sonotrode 10a mittelbar. Dazu schlägt das Projektil 62a in einem Betriebszustand auf die Verbindungshülse 84a auf.

Zur Lagerung des Projektils 62a weist die Lithotripsievorrichtung wenigstens einen Führungskanal 88a auf. Die Lithotripsievorrichtung weist ein Führungsrohr 86a auf. Das Führungsrohr 86a ist zumindest teilweise in der Ausnehmung 80a des Ultraschallhorns 12a angeordnet. Das Führungsrohr 86a definiert schließt den Führungskanal 88a ein. Alternativ könnte der Führungskanal auch von der Ausnehmung des Ultraschallhorns ausgebildet sein. Das Führungsrohr könnte im Bereich des Ultraschallhorns verschlossen sein, wodurch vorteilhaft vermieden werden kann, dass evtl. in einem pneumatischen Betrieb Druckluft in Richtung eines Patienten gelangen kann.

Die Antriebseinrichtung 64a ist pneumatisch mit dem Führungskanal 88a verbunden. An dem Führungsrohr 86a ist ein Verbindungsstutzen angeordnet. Die Antriebseinrichtung 64a ist über einen Schlauch mit dem Verbindungsstutzen verbunden. Die Antriebseinrichtung 64a überträgt Druckluftstöße auf den Führungskanal 88a, wodurch das in dem Führungskanal 88a angeordnete Projektil 62a beschleunigt wird. Die Antriebseinrichtung 64a beschleunigt das Projektil 62a innerhalb des Führungskanals 88a.

Die Lithotripsievorrichtung weist wenigstens eine Sonotrode 10a auf. Die Sonotrode 10a weist eine Haupterstreckung 82a auf. Die Haupterstreckung 82a der Sonotrode 10a entspricht der Haupterstreckung 36a des Irrigationskanals 14a. Die Sonotrode 10 ist zumindest teilweise außerhalb des Gehäuse 70a angeordnet. Im vorliegenden Fall ist die Sonotrode 10 vollständig außerhalb des Gehäuses 70a angeordnet. Die Sonotrode 10a bildet zumindest zu einem Großteil den distalen Abschnitt 68a der Lithotripsievorrichtung aus. Die Sonotrode 10a ist rohrförmig ausgebildet. Die Sonotrode 10a ist starr ausgebildet. Die Sonotrode 10a besteht zumindest teilweise aus Metall, insbesondere Stahl.

Die Sonotrode 10a ist zumindest teilweise in/an dem Ultraschallhorn 12a angeordnet. Das Ultraschallhorn 12a überträgt in einem Betriebszustand Ultraschallwellen von dem Ultraschallgenerator 74a auf die Sonotrode 10a. Dazu ist die Sonotrode 10a ist mit dem Ultraschallhorn 12a verbunden. Die Sonotrode 10a und das Ultraschallhorn 12a sind im vorliegenden Fall miteinander verschraubt. Dazu weist die Lithotripsievorrichtung eine Verbindungshülse 84a auf. Die Verbindungshülse 84a umschließt einen proximalen Endabschnitt der Sonotrode 10a. Die Sonotrode 10a ist in die Verbindungshülse 84a eingepresst. Alternativ oder zusätzlich kann die Sonotrode mit der Verbindungshülse verklebt sein. Ferner ist denkbar, dass eine solche Sonotrode mit einer solchen Verbindungshülse einstückig ausgebildet sein könnte.

Die Verbindungshülse 84a weist ein Außengewinde 96a auf. Ferner weist das Ultraschallhorn 12a ein Innengewinde 98a auf. Das Innengewinde 98a ist an einem distalen Ende des Ultraschallhorns 12a angeordnet. Das Innengewinde 98a ist an einer die Ausnehmung 80a begrenzenden Wandung angeordnet. Das Innengewinde 98a und das Außengewinde 96a greifen zu einer Verbindung der Sonotrode 10a und des Ultraschallhorns 12a ineinander ein.

Die Lithotripsievorrichtung weist wenigstens einen Irrigationskanal 14a auf. Der Irrigationskanal 14a ist zur Leitung eines Irrigat eingerichtet. Im vorliegenden Fall ist der Irrigationskanal 14a zu einer Absaugung eines Irrigats eingerichtet. Ferner ist der Irrigationskanal 14a zu einem Absaugen von Konkrementen und/oder Bruchteilen von Konkrementen, welche vom Irrigat transportiert werden eingerichtet. Alternativ könnte ein solcher Irrigationskanal auch zu einer Abgabe eines Irrigats genutzt werden. Der Irrigationskanal 14a ist zumindest teilweise von der Sonotrode 10a ausgebildet. Im vorliegenden Fall ist der Irrigationskanal 14a vollständig durch die Sonotrode 10a ausgebildet. Der Irrigationskanal 14a erstreckt sich im vorliegenden Fall nur durch die Sonotrode 10a. Eine Haupterstreckung 36a des Irrigationskanals 14a ist kleiner als eine Gesamthaupterstreckung 38a, welche sich aus einer Haupterstreckung 82a der Sonotrode 10a und einer Haupterstreckung 100a des Ultraschallhorns 12a zusammensetzt. Das Ultraschallhorn 12a ist im vorliegenden frei von einem Irrigationskanal 14a.

Ferner ist der Irrigationskanal 14a vollständig außerhalb des Gehäuses 70a angeordnet. Innerhalb des Gehäuses 70a ist kein Irrigationskanal 14a angeordnet. Alternativ kann sich ein Irrigationskanal auch zumindest teilweise durch ein Ultraschallhorn erstrecken bzw. durch ein Ultraschallhorn zumindest teilweise ausgebildet sein. Für diesen Fall ist eine Haupterstreckung 36a des Irrigationskanals 14a kleiner gewählt als eine Gesamthaupterstreckung 38a, welche sich aus der Haupterstreckung 82a der Sonotrode 10a und der Haupterstreckung 100a des Ultraschallhorns 12a zusammensetzt.

Die Sonotrode 10a weist wenigstens eine radiale Öffnung 40a auf. Die radiale Öffnung 40a ist zu einer Verbindung des Irrigationskanals 14a mit weiteren Irrigat führenden Kanälen eingerichtet. Die radiale Öffnung 40a ist an einem proximalen Endabschnitt der Sonotrode 10a angeordnet. Die radiale Öffnung 40a ist an einer Position angeordnet, an welcher sich in einem Betriebszustand ein Schwingungsbauch der Ultraschallwellen ausbildet. Die radiale Öffnung 40a ist von einer radialen Ausnehmung 80a einer Wandung der Sonotrode 10a gebildet, welche den Irrigationskanal 14a begrenzt. Ferner weist die Sonotrode 10a eine weitere radiale Öffnung 42a auf. Die weitere radiale Öffnung 42a ist zu der radialen Öffnung 40a identisch ausgebildet. Die weitere radiale Öffnung 42a ist zu der radialen Öffnung 40a entlang eines Umfangs der Sonotrode 10a versetzt angeordnet. Im vorliegenden Fall sind die radiale Öffnung 40a und die weitere radiale Öffnung 42a zueinander um 180° entlang des Umfangs der Sonotrode 10a zueinander versetzt angeordnet. Die radiale Öffnung 40a und die weitere radiale Öffnung 42a sind zueinander spiegelsymmetrisch angeordnet. Eine Spiegelsymmetrieachse der Spiegelsymmetrie entspricht dabei einer Mittelachse der Sonotrode 10a.

Innerhalb der Sonotrode10a weist der Irrigationskanal 14a wenigstens eine Abwinklung 108a auf. Die Abwinklung 108a erstreckt sich zur radialen Öffnung 40a der Sonotrode 10a hin erstreckt. Im vorliegenden Fall weist der Irrigationskanal 14a wenigstens eine weitere Abwinklung 110a innerhalb der Sonotrode 10a auf. Die weitere Abwinklung 110a erstreckt sich zur weiteren radialen Öffnung 424a der Sonotrode 10a hin. Die weiter Abwinklung 110a liegt der Abwinklung 108a spiegelsymmetrisch gegenüber. Eine Spiegelsymmetrieachse diesbezüglich entspricht einer Mittelachse oder eine Haupterstreckung 82a der Sonotrode 10a.

Die Sonotrode 10a weist ferner wenigstens eine axiale Öffnung 22a auf. Die axiale Öffnung 22a ist an einem distalen Endabschnitt der Sonotrode 10a angeordnet. Die axiale Öffnung 22a ist dazu eingerichtet, um in einem Betrieb Irrigat mit einer Umgebung auszutauschen. Zudem weist die Sonotrode 10a wenigstens eine zusätzlich radiale Öffnung 114a auf. Die zusätzliche radiale Öffnung 114a ist an einem distalen Endabschnitt der Sonotrode 10a angeordnet. Die zusätzliche radiale Öffnung 114a ist dazu eingerichtet, um in einem Betrieb Irrigat mit einer Umgebung auszutauschen.

Die Verbindungshülse 84a weist wenigstens eine zu der radialen Öffnung 40a der Sonotrode 10a kongruent angeordnete und/oder ausgebildete kongruente Öffnung 104a auf. Ferner weist die Verbindungshülse 84a eine zu der weiteren radialen Öffnung 42a der Sonotrode 10a kongruent angeordnete und/oder ausgebildete weitere kongruente Öffnung 106a auf. Die weitere kongruente Öffnung 106a ist zu der weiteren radialen Öffnung 42a der Sonotrode 10a kongruent angeordnet und/oder ausgebildet ist.

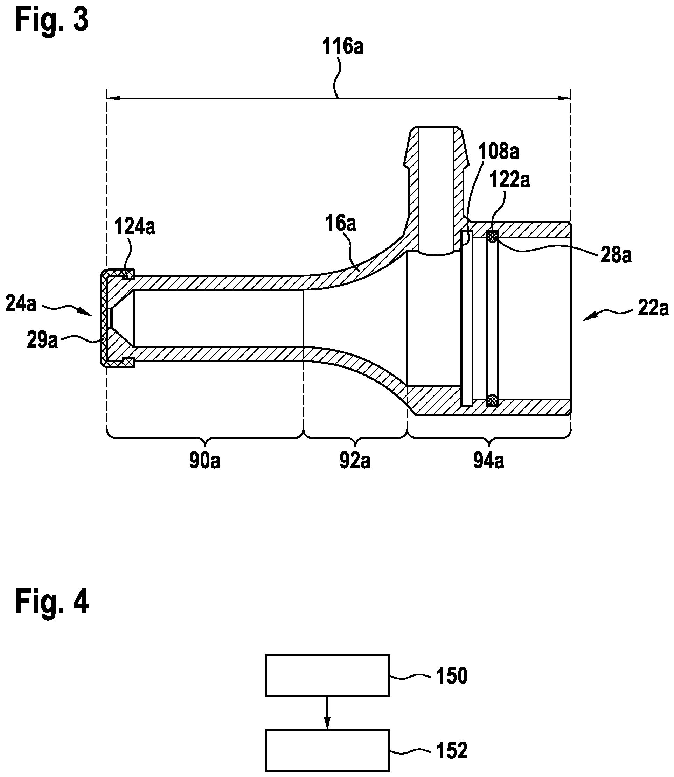

Fig. 3 zeigt eine schematische Darstellung eines Teils der Lithotripsievorrichtung mit einer Kappe 16a in einer Schnittansicht. Die Lithotripsievorrichtung weist wenigstens eine solche Kappe 16a auf. Die Kappe 16a schließt einen Spalt 20a zwischen sich, der Sonotrode 10a und dem Ultraschallhorn 12a ein (vgl. Fig. 2).

Die Kappe 16a ist als ein hohler Rotationskörper ausgebildet. Die Kappe 16a ist wenigstens teilweise trichterförmig ausgebildet. Ferner ist die Kappe 16a wenigstens abschnittsweise hohlzylinderförmig ausgebildet. Die Kappe 16a weist einen distalen Abschnitt 90a auf. Der distale Abschnitt 90a ist hohlzylinderförmig. Ferner weist die Kappe 16a einen proximale Abschnitt 94a auf. Der proximale Abschnitt 94a ist hohlzylinderförmig. Die Kappe 16a weist einen mittleren Abschnitt 92a auf. Der mittlere Abschnitt 92a ist trichterförmig.

Die Kappe 16a weist eine Haupterstreckung 116a auf. Die Haupterstreckung 116a der Kappe 16a ist kleiner als eine Haupterstreckung 82a der Sonotrode 10a. Ferner ist die Haupterstreckung 116a der Kappe 16a kleiner als eine Haupterstreckung 100a des Ultraschallhorns 12a. In einem montierten Zustand ist die Kappe 16a so angeordnet, dass diese wenigstens teilweise das Ultraschallhorn 12a und die Sonotrode 10a einschließt.

Die Kappe 16a umgibt einen distalen Endabschnitt des Ultraschallhorns 12a. Im vorliegenden Fall umgibt die Kappe 16a den distalen Endabschnitt des Ultraschallhorns 12a koaxial.

Die Kappe 16a umgibt den distalen Endabschnitt des Gehäuses 70a koaxial. Die Kappe 16a ist wenigstens abschnittsweise korrespondierend zu einer Wandung des Ultraschallhorns 12a ausgebildet. Korrespondierend zu einer konkaven Wandung des Ultraschallhorns 12a ist die Kappe 16a im mittleren Abschnitt 92a ebenfalls konkav. Die Kappe 16a ist wenigstens korrespondierend zu einer Wandung des Ultraschallhorns 12a ausgebildet.

Die Kappe 16a ist wenigstens teilweise die Sonotrode 10a koaxial umgebend angeordnet. Die Kappe 16a umgibt wenigstens einen proximalen Endabschnitt der Sonotrode 10a. Die Kappe 16a ist wenigstens die radiale Öffnung 40a der Sonotrode 10a überdeckend angeordnet. Ferner ist die Kappe 16a die weitere radiale Öffnung 42a der Sonotrode 10a umgebend angeordnet. Zudem umgibt die Kappe 16a die kongruente Öffnung 104a der Verbindungshülse 84a. Außerdem umgibt die Kappe 16a die weitere kongruente Öffnung 106a der Verbindungshülse 84a.

Die Kappe 16a weist eine axiale Öffnung 22a auf. Die axiale Öffnung 22a ist im proximalen Abschnitt 94a angeordnet. Ein Innendurchmesser der axialen Öffnung 22a entspricht einem Außendurchmesser des Gehäuses 70a. Über die axiale Öffnung 22a ist die Sonotrode 10a und das Ultraschallhorn 12a in die Kappe 16a einführbar. Alternativ könnte ein Innendurchmesser der proximalen Öffnung auch einem Außendurchmesser eines Ultraschallhorns entsprechen.

Die Kappe 16a weist eine weitere Öffnung 24a auf. Die weitere Öffnung 24a ist im distalen Abschnitt 90a angeordnet. Die weitere Öffnung 24a ist als eine axiale Öffnung 22a ausgebildet. Ein Innendurchmesser der weiteren axialen Öffnung 24a entspricht einem Außendurchmesser der Sonotrode 10a. Über die weiteren axialen Öffnung 24a ist die Sonotrode 10a durch die Kappe 16a hindurchführbar.

Die Kappe 16a liegt in einem montierten Zustand unmittelbar an dem Gehäuse 70a an. Ferner kann die Kappe 16a an dem Ultraschallhorn 12a anliegen. Alternativ oder zusätzlich ist jedoch auch denkbar, dass eine Kappe unmittelbar an einem Ultraschallhorn angeordnet ist.

Die Kappe 16a weist wenigstens einen Anschlag 26a auf. Der Anschlag 26a ist als eine Stufe ausgebildet, welche sich ringförmig entlang eines gesamten Umfangs einer Innenwandung der Kappe 16a erstreckt. Mittels des Anschlags 26a stützt sich die Kappe 16a axial an dem Gehäuse 70a ab. Ferner kann sich die Kappe 16a mittels des Anschlags 26a an dem Ultraschallhorn 12a abstützen. Der Anschlag 26a ist an einer Position angeordnet, an welcher sich in einem Betriebszustand ein Schwingungsknoten der Ultraschallwellen ausbildet. Das Ultraschallhorn 12a und/oder das Gehäuse 70a kann/können einen zu dem Anschlag 26a korrespondieren ausgebildeten korrespondierenden Anschlag 120a aufweisen, an welchem der Anschlag 26a in einem montierten Zustand anliegt.

Die Lithotripsievorrichtung weist wenigstens eine Dichtung 28a auf. Die Dichtung 28a dichtet fluiddicht den eingeschlossenen Spalt 20a zwischen der Kappe 16a, der Sonotrode 10a und dem Ultraschallhorn 12a gegenüber einer Umgebung ab. Die Dichtung 28a ist unmittelbar zwischen der Kappe 16a und dem Gehäuse 70a angeordnet. Die Dichtung 28a ist im vorliegenden Fall als ein O-Ring ausgebildet. Eine Innenwandung der Kappe 16a weist eine Nut 122a auf, in welcher die Dichtung 28a wenigstens teilweise angeordnet ist. Die Nut 122a ist ringförmig ausgebildet. Alternativ könnte eine Dichtung jedoch auch zwischen einer solchen Kappe und einem Ultraschallhorn angeordnet sein. Die Dichtung 28a ist an einer Position angeordnet, an welcher sich in einem Betriebszustand ein Schwingungsknoten der Ultraschallwellen ausbildet. Im vorliegenden Fall kontaktiert die Dichtung 28a das Gehäuse 70a an der Position, an welcher sich in dem Betriebszustand ein Schwingungsknoten der Ultraschallwellen ausbildet.

Die Lithotripsievorrichtung weist wenigstens eine weitere Dichtung 29a auf. Die weitere Dichtung 29a dichtet fluiddicht den Spalt 20a zwischen der Kappe 16a und der Sonotrode 10a ab. Die weitere Dichtung 29a kontaktiert die Sonotrode 10a. Die weitere Dichtung 29a dichtet die weitere axiale Öffnung 24a der Kappe 16a ab. Die weitere Dichtung 29a ist am distalen Ende der Kappe 16a angeordnet. Die weitere Dichtung 29a ist als eine Dichthülse ausgebildet. Die weitere Dichtung 29a ist am distalen Ende auf die Kappe 16a aufgestülpt. Die Kappe 16a weist eine weitere Nut 124a auf. Die weitere Dichtung 29a ist in der weiteren Nut 124a gehalten. Die weitere Dichtung 29a ist an einer Position angeordnet, an welcher sich in einem Betriebszustand ein Schwingungsknoten der Ultraschallwellen ausbildet. An dieser Position kontaktiert die Dichtung 28a die Sonotrode 10a.

Die Lithotripsievorrichtung weist wenigstens einen Schnellverbinder 34a auf. Der Schnellverbinder 34a ist zu einer Anordnung der Kappe 16a an der Sonotrode 10a und/oder dem Ultraschallhorn 12a eingerichtet. Der Schnellverbinder 34a ist dazu eingerichtet, eine werkzeuglos und zerstörungsfrei lösbare Verbindung herzustellen. Im vorliegenden Fall ist die Verbindung eine kraft- und/oder formschlüssige Verbindung. Der Schnellverbinder 34a ist insbesondere dazu eingerichtet, die Kappe 16a mit dem Gehäuse 70a zu verbinden. Die Kappe 16a bildet zumindest teilweise den Schnellverbinder 34a aus. Die Kappe 16a weist wenigstens ein Verbindungsteil auf. Das Verbindungsteil bildet wenigstens teilweise den Schnellverbinder 34a aus. Bei dem Verbindungsteil handelt es sich um eine Bajonettverbinderführung. Bei dem Schnellverbinder 34a kann es sich beispielsweise, um ein Bajonettverschluss, ein Schnellspannverschluss, ein Schraubverschluss oder dergleichen handeln.

Das Verbindungsteil weist einen Längsschlitz auf. Ferner weist das Verbindungsteil einem an dem Ende des Längsschlitz rechtwinklig ansetzenden Querschlitz auf. Alternativ kann es sich bei dem Verbindungsteil auch um eine Rastlasche bzw. -haken, ein Gewinde oder dergleichen handeln. Denkbar ist, dass die Kappe wenigstens zwei oder mehrere Verbindungsteile aufweist, welche insbesondere entlang einem Umfang der Kappe versetzt zueinander angeordnet seien können.

Ferner bildet das Gehäuse 70a zumindest teilweise den Schnellverbinder 34a aus. Das Gehäuse 70a weist ein zu dem Verbindungsteil korrespondierend ausgebildetes korrespondierendes Verbindungsteil auf. Das korrespondierende Verbindungsteil bildet zumindest teilweise den Schnellverbinder 34a aus. Bei dem korrespondierenden Verbindungsteil handelt es sich im vorliegenden Fall um einen Bajonettverbinderkopf. Das korrespondierende Verbindungsteil ist in den Querschlitz des Verbindungselements einführbar. Ferner ist das korrespondierende Verbindungsteil durch Verdrehen im Längsschlitz des Verbindungselements eine feste mit diesem verbindbar. Denkbar ist, dass das Gehäuse wenigstens zwei oder mehrere korrespondierende Verbindungsteile aufweist, welche insbesondere entlang einen Umfang des Gehäuses versetzt zueinander angeordnet sein können. Alternativ kann es sich bei dem korrespondierenden Verbindungsteil auch um eine Rastnase, ein Gegengewinde oder dergleichen handeln. Auch ist denkbar, dass die Kappe anstelle des Gehäuses wenigstens ein korrespondierendes Verbindungsteil aufweist und das Gehäuse anstelle der Kappe das Verbindungsteil aufweist. Für den Fall, dass mehrere Verbindungsteile und korrespondierende Verbindungsteile vorhanden sind, ist denkbar, dass die Kappe sowohl Verbindungsteile als auch korrespondierende Verbindungsteile aufweist und das Gehäuse mehrere korrespondierende Verbindungsteile und Verbindungsteile aufweist.

Die Lithotripsievorrichtung weist wenigstens einen Abführungskanal 18a auf. Der Abführungskanal 18a ist wenigstens teilweise von dem Spalt 20a, welcher zwischen der Kappe 16a, der Sonotrode 10a und dem Ultraschallhorn 12a eingeschlossen ist, gebildet. Der Abführungskanal 18a ist zu einer Kühlung der Sonotrode 10a und/oder des Ultraschallhorns 12a eingerichtet. Ferner ist der Abführungskanal 18a zu einem Abführen des Irrigats aus dem Irrigationskanal 14a eingerichtet. Der Abführungskanal 18a ist wenigstens mit dem Irrigationskanal 14a fluidtechnisch verbunden. Wenigstens über die radiale Öffnung 40a ist der Abführungskanal 18a mit dem Irrigationskanal 14a verbunden. Im vorliegenden Fall ist der Abführungskanal 18a über die radialen Öffnungen 40a, 44a sowie die kongruenten Öffnungen 104a, 106a der Verbindungshülse 84a mit dem Irrigationskanal 14a fluidtechnisch verbunden.

Der Abführungskanal 18a erstreckt sich zumindest teilweise längs der Sonotrode 10a und/oder dem Ultraschallhorn 12a. Der Abführungskanal 18a erstreckt sich zumindest teilweise quer der Sonotrode 10a und/oder dem Ultraschallhorn 12a. Der Abführungskanal 18a umgibt wenigstens teilweise einen Mantel der Sonotrode 10a und/oder des Ultraschallhorns 12a. Der Abführungskanal 18a ist zumindest teilweise röhrenförmig ausgebildet. Der Abführungskanal 18a ist zumindest teilweise trichterförmig ausgebildet. Im Bereich des distalen Abschnitts 90a der Kappe 16a ist der Abführungskanal 18a röhrenförmig. Im Bereich des mittleren Abschnitts 92a der Kappe 16a ist der Abführungskanal 18a trichterförmig.

Der Abführungskanal 18a ist fluidtechnisch mit der Fördereinrichtung 60a verbunden. Die Kappe 16a weist wenigstens einen Anschluss 30a auf. Der Abführungskanal 18a öffnet sich in Richtung des Anschlusses 30a. Über den Anschluss 30a ist der Abführungskanal 18a fluidtechnisch mit der Fördereinrichtung 60a verbunden. Im vorliegenden Fall ist der Anschluss 30a als ein Stutzen ausgebildet. Der Anschluss 30a ist separat von einer Wandung der Kappe 16a ausgebildet. Alternativ könnte der Anschluss jedoch auch mit der Wandung der Kappe einstückig ausgebildet sein.

Fig. 4 zeigt einen schematischen Ablaufplan eines beispielhaften Verfahrens zur Herstellung der Lithotripsievorrichtung.

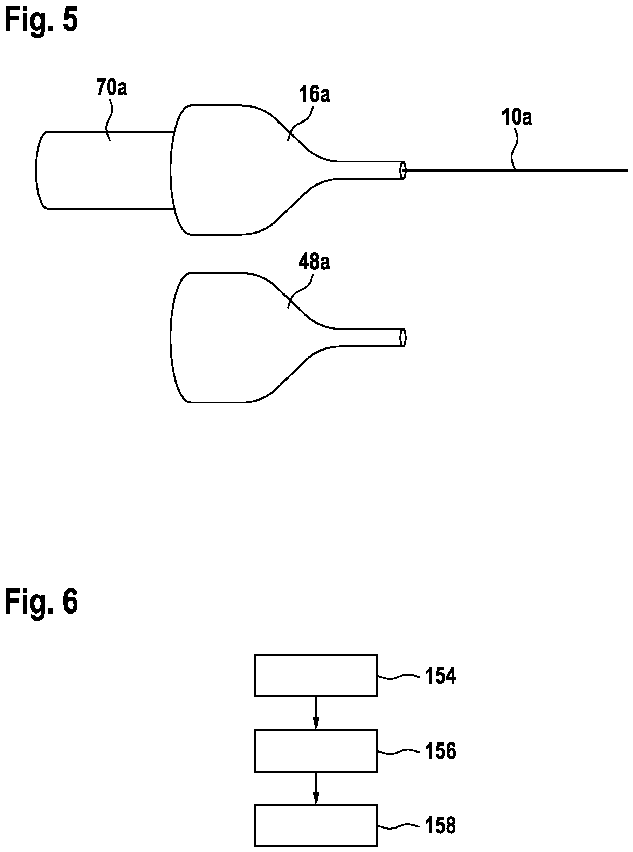

Das Verfahren umfasst wenigstens einen Verfahrensschritt 150a. In dem Verfahrensschritt 150a wird die Kappe 16a an der Sonotrode 10a bzw. dem Ultraschallhorn 12a angeordnet. Alternativ kann statt der Kappe 16a auch die zusätzliche Kappe 48a eines Bausatzes eines Lithotripters mit einer Lithotripsievorrichtung, wie beispielsweise in Fig. 5 dargestellt, Verwendung finden. Die Sonotrode 10a wird in eine axiale Öffnung 22a der Kappe 16a eingeführt. Die Kappe 16a wird in proximaler Richtung auf die Sonotrode 10a geschoben. Die Kappe 16a wird solange geschoben, bis der Anschlag 26a der Kappe 16a an der Ultraschallhorn 12a anliegt. Ferner wird die Kappe 16a soweit geschoben, bis deren Anschlag 26a an dem Gehäuse 70a anliegt.

Das Verfahren umfasst wenigstens einen weiteren Verfahrensschritt 152a. In dem Verfahrensschritt 152a wird die Kappe 16a befestigt. Dazu wird der Schnellverbinder 34a genutzt. Das Verbindungsteil und das korrespondierende Verbindungsteil des Schnellverbinders 34a werden miteinander fest verbunden. Dazu wird das korrespondierende Verbindungsteil in den Querschlitz des Verbindungselements eingeführt. Ferner wird das korrespondierende Verbindungsteil im Längsschlitz des Verbindungselements verdreht.

Im Anschluss an dieses Verfahren kann die Lithotripsievorrichtung mit weiteren Komponenten des Lithotripsiesystems verbunden werden. Beispielsweise kann die Antriebseinrichtung mit dem Führungskanal verbunden werden. Ferner kann die Fördereinheit mit dem Abführungskanal verbunden werden. Um die Lithotripsievorrichtung zu demontieren, insbesondere zu einer Reinigung, werden die obigen Verfahrensschritte in umgekehrter Reihenfolge ausgebführt.

Fig. 6 zeigt einen schematischen Ablaufplan eines beispielhaften Verfahrens zum Betrieb der Lithotripsievorrichtung. Bei dem Verfahren handelt es sich um ein Testverfahren, um beispielsweise einen Ablauf oder eine Dauer eines chirurgischen Eingriffs zu testen.

Das Verfahren umfasst wenigstens einen Verfahrensschritt 154a. In dem Verfahrensschritt 154a wird die Lithotripsievorrichtung aktiviert. Die Aktivierung erfolgt durch Bedienung des Betätigungsmittels 58a. Die Steuerelektronik des Steuergeräts 52a startet die Antriebseinheit. Die Steuerelektronik des Steuergeräts 52a startet die Fördereinrichtung 60a. Die Steuerelektronik des Steuergeräts 52a startet den Ultraschallgenerator 74a.

Das Verfahren umfasst einen weiteren Verfahrensschritt 156a. In dem weiteren Verfahrensschritt 156a wird die Sonotrode 10a mit einem Konkrement in Kontakt gebracht. Bei dem Konkrement kann es sich um ein Probekörper handeln, welcher in seinen Eigenschaften Nierensteinen oder sonstigen Körpersteinen ähnelt. Die von dem Ultraschallgenerator 74a bereitgestellten Ultraschallwellen werden von dem Ultraschallhorn 12a auf die Sonotrode 10a übertragen. Ferner werden die Ultraschallwellen von dem Ultraschallhorn 12a fokussiert. Die Sonotrode 10a überträgt die Ultraschallwellen wiederum auf das Konkrement. Ferner wird durch die Antriebseinrichtung 64a das Projektil 62a im Führungskanal 88a beschleunigt. Das Projektil 62a überträgt seinen Stoßimpuls auf die Sonotrode 10a. Die Sonotrode 10a gibt den Stoßimpuls auf das Konkrement ab.

Das Verfahren umfasst einen weiteren Verfahrensschritt 158a. In dem weiteren Verfahrensschritt 158a wird mittels der Fördereinrichtung 60a ein Irrigat, mit welchem das Konkrement umspült wird, abgesaugt. Die Fördereinrichtung 60a erzeugt einen Unterdruck, wodurch das Irrigat in den Irrigationskanal 14a gefördert wird. Das Irrigat fließt durch die axiale Öffnung 22a der Sonotrode 10a in den Irrigationskanal 14a. Ferner fließt das Irrigat durch die zusätzliche radiale Öffnung 114a in den Irrigationskanal 14a. Beispielsweise fließt das Irrigat zu einem größeren Teil durch die zusätzliche radiale Öffnung 114a, wenn die axiale Öffnung 22a durch das an die Sonotrode 10a axial anliegende Konkrement versperrt wird. Das Irrigat transportiert Bruchstücke des mit Ultraschall und/oder Stoßimpulsen behandelten Konkrements mit ab. Mittels des Abführungskanals 18a wird das Irrigat abgeführt. Das Irrigat wird mittels der Fördereinrichtung 60a von dem Irrigationskanal 14a in den Abführungskanal 18a transportiert. Das Irrigat fließt durch radiale Öffnungen 40a, 42a des Irrigationskanals 14a in einen Spalt 20a zwischen der Kappe 16a, der Sonotrode 10a und dem Ultraschallhorn 12a. Dieser Spalt 20a bildet im Betrieb den Abführungskanal 18a aus. Ferner wird das Irrigat aus dem Abführungskanal 18a transportiert. Das Irrigat wird über den Anschluss 30a der Kappe 16a aus dem Abführungskanal 18a transportiert.

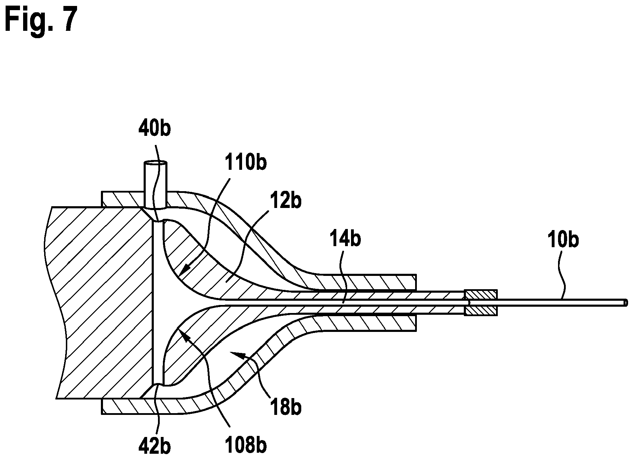

In der Fig. 7 ist ein weiteres offenbarungsgemäße Ausführungsbeispiel gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Figuren 1 bis 6 verwiesen wird. Auch sollen sämtliche Kombinationen der hier genannten Ausführungsbeispiele als mit offenbart gelten. Zur Unterscheidung der Ausführungsbeispiele ist der Buchstabe a den Bezugszeichen des Ausführungsbeispiels in den Figuren 1 bis 6 nachgestellt. In den Ausführungsbeispiel der Figur 7 ist der Buchstabe a durch die Buchstaben b ersetzt.

Fig. 7 zeigt eine schematische Darstellung weiter Ausgestaltung einer Lithotripsievorrichtung in einer Schnittansicht. Die vorliegende Lithotripsievorrichtung unterscheidet sich von der vorhergehenden im Wesentlichen durch eine Ausgestaltung eines Irrigationskanals 14b der Lithotripsievorrichtung. Im vorliegenden Fall erstreckt sich der Irrigationskanal 14b sowohl durch eine Sonotrode 10b der Lithotripsievorrichtung als auch durch ein Ultraschallhorn 12b der Lithotripsievorrichtung.

Innerhalb des Ultraschallhorns 12b weist der Irrigationskanal 14b wenigstens eine Abwinklung 108b auf. Entlang der Abwinklung 108b knickt der Irrigationskanal14b ab. Im vorliegenden Fall knickt der Irrigationskanal 14b um 90° ab. Ferner könnte der Irrigationskanal auch um einen Winkel von wenigstens 0° und höchstens 90° abknicken. Ferner könnte der Irrigationskanal innerhalb der Sonotrode eine Abwinklung 108b aufweisen. Ferner weist der Irrigationskanal 14b wenigstens eine weitere Abwinklung 110b auf. Die weitere Abwinklung 110b liegt der Abwinklung 108b spiegelsymmetrisch gegenüber. Im vorliegenden Fall ist eine Spiegelsymmetrieachse gleich einer Mittelachse der Sonotrode 10b.

Das Ultraschallhorn 12b weist wenigstens eine radiale Öffnung 40b auf. Die Abwinklung 108b erstreckt sich zur radialen Öffnung 40b hin. Derart öffnet dich der Irrigationskanal 14b in Richtung eines Abführungskanals 18b der Lithotripsievorrichtung. Ferner weist das Ultraschallhorn 12b wenigstens eine weitere radiale Öffnung 42b auf. Die weitere radiale Öffnung 42b liegt der radialen Öffnung 40b spiegelsymmetrisch gegenüber. Im vorliegenden Fall ist eine Spiegelsymmetrieachse gleich einer Mittelachse der Ultraschallhorns 12b.

| | | | |
|---|---|---|---|
| 10 | Sonotrode | 68 | Distaler Abschnitt |
| 12 | Ultraschallhorn | 70 | Gehäuse |
| 14 | Irrigationskanal | 72 | Handhabe |
| 16 | Kappe | 74 | Ultraschallgenerator |
| 18 | Abführungskanal | 78 | Ultraschallaktor |
| 20 | Spalt | 80 | Ausnehmung |
| 22 | Axiale Öffnung | 82 | Haupterstreckung der Sonotrode |
| 24 | Weitere axiale Öffnung | 84 | Verbindungshülse |
| 26 | Anschlag | 86 | Führungsrohr |
| 28 | Dichtung | 88 | Führungskanal |
| 29 | Weitere Dichtung | 90 | Distaler Abschnitt |
| 30 | Anschluss | 92 | Mittlerer Abschnitt |
| 34 | Schnellverbinder | 94 | Proximaler Abschnitt |
| 36 | Haupterstreckung des Irrigationskanals | 96 | Außengewinde |
| | | 98 | Innengewinde |
| 38 | Gesamthaupterstreckung | | |
| 40 | Radiale Öffnung | 100 | Haupterstreckung des Ultraschallhorns |
| 42 | Weitere radiale Öffnung | 104 | Kongruente Öffnung |
| 46 | Lithotripter | 106 | Weitere Kongruente Öffnung |
| 48 | Zusätzliche Kappe | 108 | Abwinklung |
| 50 | Lithotripsiesystem | 110 | Weitere Abwinklung |
| 52 | Steuergerät | 114 | Zusätzliche radiale Öffnung |
| 58 | Betätigungsmittel | 116 | Haupterstreckung der Kappe |
| 60 | Fördereinrichtung | 120 | Korrespondierender Anschlag |
| 62 | Projektil | 122 | Nut |
| 64 | Antriebseinrichtung | 124 | Weitere Nut |
| 66 | Proximaler Abschnitt | 150 | Verfahrensschritt |

| | | | |
|---|---|---|---|
| 152 | Verfahrensschritt | 156 | Verfahrensschritt |
| 154 | Verfahrensschritt | 158 | Verfahrensschritt |

## Patentansprüche

1. Lithotripsievorrichtung, insbesondere intrakorporale Lithotripsievorrichtung,
mit wenigstens einer Sonotrode (10a, 10b), welche zur Beaufschlagung von Konkrementen zumindest mit Ultraschallwellen eingerichtet ist, mit wenigstens einem Ultraschallhorn (12a, 12b), welches zumindest zur Fokussierung und/oder Übertragung von Ultraschallwellen auf die Sonotrode (10a, 10b) eingerichtet ist, mit wenigstens einem Irrigationskanal (14a, 14b), welcher zur Leitung eines Irrigats eingerichtet ist und welcher sich zumindest teilweise durch die Sonotrode (10a, 10b) und/oder das Ultraschallhorn (12a, 12b) erstreckt, mit wenigstens einer Kappe (16a), welche die Sonotrode (10a, 10b) und/oder das Ultraschallhorn (12a, 12b) zumindest teilweise umgebend angeordnet ist, sowie mit wenigstens einem Abführungskanal (18a, 18b), welcher fluidtechnisch zu einem Abführen des Irrigats mit dem Irrigationskanal (14a, 14b) verbunden ist, **dadurch gekennzeichnet, dass** der Abführungskanal (18a, 18b) wenigstens teilweise von einem zwischen der Kappe (16a) und der Sonotrode (10a, 10b) und/oder dem Ultraschallhorn (12a, 12b) eingeschlossenen Spalt (20a) ausgebildet ist und zu einer Kühlung der Sonotrode (10a, 10b) und/oder des Ultraschallhorns (12a, 12b) mittels des abgeführten Irrigats eingerichtet ist.

2. Lithotripsievorrichtung nach einem der vorhergehenden Ansprüche, **dadurch gekennzeichnet, dass** die Kappe (16a) die Sonotrode (10a, 10b) und/oder das Ultraschallhorn (12a, 12b) koaxial umgebend angeordnet ist.

3. Lithotripsievorrichtung nach einem der vorhergehenden Ansprüche, **dadurch gekennzeichnet, dass** die Kappe (16a) korrespondierend zu der Sonotrode (10a, 10b) und/oder dem Ultraschallhorn (12a, 12b) ausgebildet ist.

4. Lithotripsievorrichtung nach einem der vorhergehenden Ansprüche, **dadurch gekennzeichnet, dass** die Kappe (16a) wenigstens eine axiale Öffnung (22a) aufweist, durch welche sich die Sonotrode (10a, 10b) hindurch erstreckt.

5. Lithotripsievorrichtung nach einem der vorhergehenden Ansprüche, **dadurch gekennzeichnet, dass** die Kappe (16a) wenigstens eine weitere axiale Öffnung (24a) aufweist, durch welche sich das Ultraschallhorn (12a, 12b) hindurch erstreckt.

6. Lithotripsievorrichtung nach einem der vorhergehenden Ansprüche, **dadurch gekennzeichnet, dass** die Kappe (16a) zu einer zumindest teilweise die Sonotrode (10a, 10b) und/oder das Ultraschallhorn (12a, 12b) umgebenden Anordnung wenigstens einen Anschlag (26a) aufweist.

7. Lithotripsievorrichtung nach Anspruch 6, **dadurch gekennzeichnet, dass** der Anschlag (26a) ist an einer Position innerhalb eines Bereichs, wobei der Bereich entlang der Haupterstreckungsrichtung der Sonotrode gemessen eine Länge von höchstens einem Viertel, vorzugsweise höchstens einem Achtel und besonders bevorzugt höchstens einem Sechzehntel einer Wellenlänge des Ultraschalls entspricht, um einen Schwingungsknoten der Ultraschallwellen angeordnet oder am Ort eines Schwingungsknoten der Ultraschallwellen selbst angeordnet

8. Lithotripsievorrichtung nach einem der vorhergehenden Ansprüche, **gekennzeichnet durch** wenigstens eine Dichtung (28a), welche einen den Abführungskanal (18a, 18b) wenigstens teilweise ausbildenden Spalt (20a) zwischen der Kappe (16a) und der Sonotrode (10a, 10b) und/oder des Ultraschallhorns (12a, 12b) gegenüber einer Umgebung fluiddicht abdichtet.

9. Lithotripsievorrichtung nach Anspruch 8, **dadurch gekennzeichnet, dass** die Dichtung (28a) ist an einer Position innerhalb eines Bereichs, wobei der Bereich entlang der Haupterstreckungsrichtung der Sonotrode gemessen eine Länge von höchstens einem Viertel, vorzugsweise höchstens einem Achtel und besonders bevorzugt höchstens einem Sechzehntel einer Wellenlänge des Ultraschalls entspricht, um einen Schwingungsknoten der Ultraschallwellen angeordnet oder am Ort eines Schwingungsknoten der Ultraschallwellen selbst angeordnet

10. Lithotripsievorrichtung nach einem der vorhergehenden Ansprüche, **dadurch gekennzeichnet, dass** die Kappe (16a) wenigste einen mit dem Abführungskanal (18a, 18b) fluidtechnisch verbundenen Anschluss (30a) aufweist.

11. Lithotripsievorrichtung nach einem der vorhergehenden Ansprüche, **gekennzeichnet durch** wenigstens einen zu einer zumindest teilweise die Sonotrode (10a, 10b) und/oder das Ultraschallhorn (12a, 12b) umgebenden Anordnung der Kappe (16a) eingerichteten Schnellverbinder (34a), wie beispielsweise ein Bajonettverbinder, ein Schnellspannverbinder, ein Schraubverbinder oder dergleichen, welcher wenigstens teilweise von der Kappe (16a) ausgebildet ist.

12. Lithotripsievorrichtung nach einem der vorhergehenden Ansprüche, **dadurch gekennzeichnet, dass** die Kappe (16a) zumindest teilweise transparent ausgebildet ist.

13. Lithotripsievorrichtung nach einem der vorhergehenden Ansprüche, **dadurch gekennzeichnet, dass** die Kappe (16a) als ein Einwegbauteil eingerichtet ist.

14. Lithotripsievorrichtung nach einem der vorhergehenden Ansprüche, **dadurch gekennzeichnet, dass** der Abführungskanal (18a, 18b) die Sonotrode (10a, 10b) und/oder das Ultraschallhorn (12a, 12b) entlang ihres/deren Umfangs umgibt.

15. Lithotripsievorrichtung nach einem der vorhergehenden Ansprüche, **dadurch gekennzeichnet, dass** der Abführungskanal (18a, 18b) wenigstens abschnittsweise trichterförmig ausgebildet ist.

16. Lithotripsievorrichtung nach einem der vorhergehenden Ansprüche, **dadurch gekennzeichnet, dass** der Irrigationskanals (14a, 14b) eine Haupterstreckung (36a) aufweist und die Sonotrode (10a, 10b) und das Ultraschallhorn (12a, 12b) zusammen eine Gesamthaupterstreckung (38a) aufweisen, wobei die Haupterstreckung (36a) des Irrigationskanals (14a, 14b) kleiner ist als die Gesamthaupterstreckung (38a) der Sonotrode (10a, 10b) und des Ultraschallhorns (12a, 12b).

17. Lithotripsievorrichtung nach einem der vorhergehenden Ansprüche, **dadurch gekennzeichnet, dass** die Sonotrode (10a, 10b) und/oder das Ultraschallhorn (12a, 12b) zu einer fluidtechnischen Verbindung des Irrigationskanal (14a, 14b) mit dem Abführungskanal (18a, 18b) wenigstens eine radiale Öffnung (40a, 40b, 42a, 42b, 114a) aufweist.

18. Lithotripsievorrichtung nach Anspruch 17, **dadurch gekennzeichnet, dass** die radiale Öffnung (40a, 40b, 42a, 42b, 114a) der Sonotrode (10a, 10b) und/oder des Ultraschallhorns (12a, 12b) ist an einer Position innerhalb eines Bereichs, wobei der Bereich entlang der Haupterstreckungsrichtung der Sonotrode gemessen eine Länge von höchstens einem Viertel, vorzugsweise höchstens einem Achtel und besonders bevorzugt höchstens einem Sechzehntel einer Wellenlänge des Ultraschalls entspricht, um einen Schwingungsbauch der Ultraschallwellen angeordnet oder am Ort eines Schwingungsbauch der Ultraschallwellen selbst angeordnet.

19. Lithotripsievorrichtung nach Anspruch 17 oder 18, **dadurch gekennzeichnet, dass** die Kappe (16a) die radiale Öffnung (40a, 40b, 42a, 42b, 114a) der Sonotrode (10a, 10b) und/oder des Ultraschallhorns (12a, 12b) überdeckend angeordnet ist.

20. Lithotripsievorrichtung nach einem der vorhergehenden Ansprüche, **dadurch gekennzeichnet, dass** der Irrigationskanal (14a, 14b) innerhalb der Sonotrode (10a, 10b) und/oder des Ultraschallhorns (12a, 12b) wenigstens eine Abwinklung (108b) aufweist.

21. Lithotripter (46a) mit wenigstens einer Lithotripsievorrichtung nach einem der vorhergehenden Ansprüche.

22. Bausatz für einen Lithotripter (46a) mit wenigstens einer Lithotripsievorrichtung nach einem der Ansprüche 1 bis 20 sowie mit wenigstens einer zusätzlichen Kappe (48a).

23. Lithotripsiesystem (50a) mit einem Lithotripter (46a) nach Anspruch 21 und mit wenigstens einem Steuergerät (52a), welches zumindest zu einer Steuerung wenigstens des Lithotripters (46a) eingerichtet ist.

## Claims

1. Lithotripsy device, in particular an intracorporeal lithotripsy device, having at least one sonotrode (10a, 10b) configured to apply at least ultrasonic waves to concretions, having at least one ultrasonic horn (12a, 12b) configured at least to focus and/or transfer ultrasonic waves on/to the sonotrode (10a, 10b), having at least one irrigation channel (14a, 14b) configured to conduct an irrigation fluid and extending at least partially through the sonotrode (10a, 10b) and/or the ultrasonic horn (12a, 12b), having at least one cap (16a) arranged so as to at least partially surround the sonotrode (10a, 10b) and/or the ultrasonic horn (12a, 12b), and having at least one discharge channel (18a, 18b) fluid-connected to the irrigation channel (14a, 14b) for the purpose of discharging the irrigation fluid, **characterized in that** the discharge channel (18a, 18b) is formed at least in part by a gap (20a) enclosed between the cap (16a) of the sonotrode (10a, 10b) and/or the ultrasonic horn (12a, 12b) and is configured to cool the sonotrode (10a, 10b) and/or the ultrasonic horn (12a, 12b) by means of the discharged irrigation fluid.

2. Lithotripsy device according to any of the preceding claims, **characterized in that** the cap (16a) is arranged so as to coaxially surround the sonotrode (10a, 10b) and/or the ultrasonic horn (12a, 12b).

3. Lithotripsy device according to any of the preceding claims, **characterized in that** the cap (16a) is formed in a manner corresponding to the sonotrode (10a, 10b) and/or the ultrasonic horn (12a, 12b).

4. Lithotripsy device according to any of the preceding claims, **characterized in that** the cap (16a) has at least one axial opening (22a) through which the sonotrode (10a, 10b) extends.

5. Lithotripsy device according to any of the preceding claims, **characterized in that** the cap (16a) has at least one further axial opening (24a) through which the ultrasonic horn (12a, 12b) extends.

6. Lithotripsy device according to any of the preceding claims, **characterized in that** the cap (16a) has at least one stop (26a) for an arrangement at least partially surrounding the sonotrode (10a, 10b) and/or the ultrasonic horn (12a, 12b).

7. Lithotripsy device according to Claim 6, **characterized in that** the stop (26a) is at a position within a region, the region as measured along the main direction of extent of the sonotrode corresponding to a length of no more than one quarter, preferably no more than one eighth and particularly preferably no more than one sixteenth of a wavelength of the ultrasound, arranged about a node of the ultrasonic waves or arranged at the location of a node of the ultrasonic waves themselves.

8. Lithotripsy device according to any of preceding claims, **characterized by** at least one seal (28a) which fluid-tightly seals a gap (20a) which at least partially forms the discharge channel (18a, 18b) and is located between the cap (16a) and the sonotrode (10a, 10b) and/or the ultrasonic horn (12a, 12b) vis-à-vis surroundings.

9. Lithotripsy device according to Claim 8, **characterized in that** the seal (28a) is at a position within a region, the region as measured along the main direction of extent of the sonotrode corresponding to a length of no more than one quarter, preferably no more than one eighth and particularly preferably no more than one sixteenth of a wavelength of the ultrasound, arranged about a node of the ultrasonic waves or arranged at the location of a node of the ultrasonic waves themselves.

10. Lithotripsy device according to any of the preceding claims, **characterized in that** the cap (16a) has at least one connector (30a) fluid-connected to the discharge channel (18a, 18b).

11. Lithotripsy device according to any of the preceding claims, **characterized by** at least one quick connector (34a), for example a bayonet connector, a quick coupling, a screw connector or the like, which is configured for an arrangement of the cap (16a) at least partially surrounding the sonotrode (10a, 10b) and/or the ultrasonic horn (12a, 12b) and which is formed at least in part by the cap (16a).

12. Lithotripsy device according to any of the preceding claims, **characterized in that** the cap (16a) has an at least partially transparent design.

13. Lithotripsy device according to any of the preceding claims, **characterized in that** the cap (16a) is configured as a disposable component.

14. Lithotripsy device according to any of the preceding claims, **characterized in that** the discharge channel (18a, 18b) surrounds the sonotrode (10a, 10b) and/or the ultrasonic horn (12a, 12b) along the circumference thereof.

15. Lithotripsy device according to any of the preceding claims, **characterized in that** the discharge channel (18a, 18b) has a funnel-shaped embodiment, at least in sections.

16. Lithotripsy device according to any of the preceding claims, **characterized in that** the irrigation channel (14a, 14b) has a main extent (36a) and the sonotrode (10a, 10b) and the ultrasonic horn (12a, 12b) together have an overall main extent (38a), with the main extent (36a) of the irrigation channel (14a, 14b) being smaller than the overall main extent (38a) of the sonotrode (10a, 10b) and ultrasonic horn (12a, 12b).

17. Lithotripsy device according to any of the preceding claims, **characterized in that** the sonotrode (10a, 10b) and/or the ultrasonic horn (12a, 12b) have at least one radial opening (40a, 40b, 42a, 42b, 114a) for a fluidconnection of the irrigation channel (14a, 14b) to the discharge channel (18a, 18b).

18. Lithotripsy device according to Claim 17, **characterized in that** the radial opening (40a, 40b, 42a, 42b, 114a) of the sonotrode (10a, 10b) and/or of the ultrasonic horn (12a, 12b) is at a position within a region, the region as measured along the main direction of extent of the sonotrode corresponding to a length of no more than one quarter, preferably no more than one eighth and particularly preferably no more than one sixteenth of a wavelength of the ultrasound, arranged about an antinode of the ultrasonic waves or arranged at the location of an antinode of the ultrasonic waves themselves.

19. Lithotripsy device according to Claim 17 or 18, **characterized in that** the cap (16a) is arranged to cover the radial opening (40a, 40b, 42a, 42b, 114a) of the sonotrode (10a, 10b) and/or of the ultrasonic horn (12a, 12b) .

20. Lithotripsy device according to any of the preceding claims, **characterized in that** the irrigation channel (14a, 14b) has at least one angled section (108b) within the sonotrode (10a, 10b) and/or the ultrasonic horn (12a, 12b) .

21. Lithotripter (46a) having at least one lithotripsy device according to any of the preceding claims.

22. Kit for a lithotripter (46a), having at least one lithotripsy device according to any of Claims 1 to 20 and having at least one additional cap (48a).

23. Lithotripsy system (50a) having a lithotripter (46a) according to Claim 21 and having at least one controller (52a), configured at least to control at least the lithotripter (46a).

## Revendications

1. Dispositif de lithotripsie, notamment dispositif de lithotripsie intracorporelle, comprenant au moins une sonotrode (10a, 10b) qui est conçue pour soumettre des concrétions à au moins des ondes ultrasonores, au moins un cornet à ultrasons (12a, 12b) qui est conçu au moins pour focaliser et/ou transmettre des ondes ultrasonores à la sonotrode (10a, 10b), au moins un conduit d'irrigation (14a, 14b) qui est conçu pour conduire un irrigant et qui s'étend au moins partiellement à travers la sonotrode (10a, 10b) et/ou le cornet à ultrasons (12a, 12b), au moins un capuchon (16a) qui est disposé de manière à entourer au moins partiellement la sonotrode (10a, 10b) et/ou le cornet à ultrasons (12a, 12b), et au moins un conduit d'évacuation (18a, 18b) qui est relié fluidiquement au conduit d'irrigation (14a, 14b) afin d'évacuer l'irrigant, **caractérisé en ce que** le conduit d'évacuation (18a, 18b) est au moins partiellement formé par un espace (20a) enfermé entre le capuchon (16a) et la sonotrode (10a, 10b) et/ou le cornet à ultrasons (12a, 12b) et est conçu pour refroidir la sonotrode (10a, 10b) et/ou le cornet à ultrasons (12a, 12b) au moyen de l'irrigant évacué.

2. Dispositif de lithotripsie selon l'une des revendications précédentes, **caractérisé en ce que** le capuchon (16a) est disposé de manière à entourer coaxialement la sonotrode (10a, 10b) et/ou le cornet à ultrasons (12a, 12b).

3. Dispositif de lithotripsie selon l'une des revendications précédentes, **caractérisé en ce que** le capuchon (16a) est conçu de manière à correspondre à la sonotrode (10a, 10b) et/ou au cornet à ultrasons (12a, 12b) .

4. Dispositif de lithotripsie selon l'une des revendications précédentes, **caractérisé en ce que** le capuchon (16a) comporte au moins une ouverture axiale (22a) à travers laquelle s'étend la sonotrode (10a, 10b).

5. Dispositif de lithotripsie selon l'une des revendications précédentes, **caractérisé en ce que** le capuchon (16a) comporte au moins une autre ouverture axiale (24a) à travers laquelle s'étend le cornet à ultrasons (12a, 12b).

6. Dispositif de lithotripsie selon l'une des revendications précédentes, **caractérisé en ce que** le capuchon (16a) comporte au moins une butée (26a) destinée à venir entourer au moins partiellement la sonotrode (10a, 10b) et/ou le cornet à ultrasons (12a, 12b).

7. Dispositif de lithotripsie selon la revendication 6, **caractérisé en ce que** la butée (26a) est située à une position à l'intérieur d'une zone, la zone, mesurée suivant la direction d'extension principale de la sonotrode, correspondant à une longueur d'au plus un quart, de préférence d'au plus un huitième et de manière particulièrement préférée d'au plus un seizième d'une longueur d'onde des ultrasons, en étant disposée autour d'un noeud d'oscillation des ondes ultrasonores ou à l'emplacement même d'un noeud d'oscillation des ondes ultrasonores.

8. Dispositif de lithotripsie selon l'une des revendications précédentes, **caractérisé par** au moins une garniture d'étanchéité (28a) qui réalise l'étanchéité aux fluides d'un espace (20a) formant au moins partiellement le conduit d'évacuation (18a, 18b) et ménagé entre le capuchon (16a) et la sonotrode (10a, 10b) et/ou le cornet à ultrasons (12a, 12b) par rapport à un environnement.

9. Dispositif de lithotripsie selon la revendication 8, **caractérisé en ce que** la garniture d'étanchéité (28a) est située à une position à l'intérieur d'une zone, la zone, mesurée suivant la direction d'extension principale de la sonotrode, correspondant à une longueur d'au plus un quart, de préférence d'au plus un huitième et de manière particulièrement préférée d'au plus un seizième d'une longueur d'onde des ultrasons, en étant disposée autour d'un noeud d'oscillation des ondes ultrasonores ou à l'emplacement même d'un noeud d'oscillation des ondes ultrasonores.

10. Dispositif de lithotripsie selon l'une des revendications précédentes, **caractérisé en ce que** le capuchon (16a) comporte au moins un raccord (30a) relié fluidiquement au conduit d'évacuation (18a, 18b).

11. Dispositif de lithotripsie selon l'une des revendications précédentes, **caractérisé par** au moins un connecteur rapide (34a), tel qu'un connecteur à baïonnette, un connecteur à serrage rapide, un connecteur à vis ou similaire, qui est au moins partiellement formé par le capuchon (16a) et qui est conçu en agencement du capuchon (16a) entourant au moins partiellement la sonotrode (10a, 10b) et/ou le cornet à ultrasons (12a, 12b) .

12. Dispositif de lithotripsie selon l'une des revendications précédentes, **caractérisé en ce que** le capuchon (16a) est au moins partiellement transparent.

13. Dispositif de lithotripsie selon l'une des revendications précédentes, **caractérisé en ce que** le capuchon (16a) est conçu comme un composant jetable.

14. Dispositif de lithotripsie selon l'une des revendications précédentes, **caractérisé en ce que** le conduit d'évacuation (18a, 18b) entoure la sonotrode (10a, 10b) et/ou le cornet à ultrasons (12a, 12b) suivant sa/leur circonférence.

15. Dispositif de lithotripsie selon l'une des revendications précédentes, **caractérisé en ce que** le conduit d'évacuation (18a, 18b) est conçu au moins par portions en forme d'entonnoir.

16. Dispositif de lithotripsie selon l'une des revendications précédentes, **caractérisé en ce que** le conduit d'irrigation (14a, 14b) présente une extension principale (36a) et la sonotrode (10a, 10b) et le cornet à ultrasons (12a, 12b) présentent conjointement une extension principale totale (38a), l'extension principale (36a) du conduit d'irrigation (14a, 14b) étant plus petite que l'extension principale totale (38a) de la sonotrode (10a, 10b) et du cornet à ultrasons (12a, 12b) .

17. Dispositif de lithotripsie selon l'une des revendications précédentes, **caractérisé en ce que** la sonotrode (10a, 10b) et/ou le cornet à ultrasons (12a, 12b) comporte(nt) au moins une ouverture radiale (40a, 40b, 42a, 42b, 114a) pour relier fluidiquement le conduit d'irrigation (14a, 14b) au conduit d'évacuation (18a, 18b) .

18. Dispositif de lithotripsie selon la revendication 17, **caractérisé en ce que** l'ouverture radiale (40a, 40b, 42a, 42b, 114a) de la sonotrode (10a, 10b) et/ou du cornet à ultrasons (12a, 12b) est à une position à l'intérieur d'une zone, la zone, mesurée suivant la direction d'extension principale de la sonotrode, correspondant à une longueur d'au plus un quart, de préférence d'au plus un huitième et de manière particulièrement préférée d'au plus un seizième d'une longueur d'onde des ultrasons, en étant disposée autour d'un ventre des ondes ultrasonores ou à l'emplacement même d'un ventre des ondes ultrasonores.

19. Dispositif de lithotripsie selon la revendication 17 ou 18, **caractérisé en ce que** le capuchon (16a) recouvre l'ouverture radiale (40a, 40b, 42a, 42b, 114a) de la sonotrode (10a, 10b) et/ou du cornet à ultrasons (12a, 12b) .

20. Dispositif de lithotripsie selon l'une des revendications précédentes, **caractérisé en ce que** le conduit d'irrigation (14a, 14b) comporte au moins un coude (108b) à l'intérieur de la sonotrode (10a, 10b) et/ou du cornet à ultrasons (12a, 12b).

21. Lithotripteur (46a) comprenant au moins un dispositif de lithotripsie selon l'une des revendications précédentes.

22. Kit destiné à un lithotripteur (46a) et comprenant au moins un dispositif de lithotripsie selon l'une des revendications 1 à 20 et au moins un capuchon supplémentaire (48a).

23. Système de lithotripsie (50a) comprenant un lithotripteur (46a) selon la revendication 21 et au moins un dispositif de commande (52a) qui est conçu au moins pour commander au moins le lithotripteur (46a).
